(19) Europäisches Patentamt
European Patent Office
Office européen des brevets

(11) EP 3 431 609 B1

(12) **EUROPEAN PATENT SPECIFICATION**

(45) Date of publication and mention of the grant of the patent:
**12.02.2020 Bulletin 2020/07**

(51) Int Cl.:
***C12Q 1/6886*** *(2018.01)*

(21) Application number: **17181646.5**

(22) Date of filing: **17.07.2017**

(54) **METHOD OF DIAGNOSIS OF COLORECTAL CANCER**

VERFAHREN ZUR DIAGNOSE VON KOLOREKTALKREBS

PROCÉDÉ DE DIAGNOSTIC DU CANCER COLORECTAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**23.01.2019 Bulletin 2019/04**

(73) Proprietors:
- **Masarykova Univerzita**
  **60177 Brno (CZ)**
- **Masarykuv Onkologicky Ustav**
  **656 53 Brno (CZ)**

(72) Inventors:
- **Slaby, Ondrej**
  **60200 Brno (CZ)**
- **Vychytilova, Petra**
  **62800 Brno (CZ)**
- **Svoboda, Marek**
  **66467 Syrovice (CZ)**
- **Sachlova, Milana**
  **60200 Brno (CZ)**

(74) Representative: **Hartvichova, Katerina et al**
**HARBER IP s.r.o.**
**Dukelskych hrdinu 567/52**
**170 00 Praha 7 (CZ)**

(56) References cited:
- **PETRA VYCHYTILOVA-FALTEJSKOVA ET AL: "Serum-based microRNA signatures in early diagnosis and prognosis prediction of colon cancer", CARCINOGENESIS., vol. 37, no. 10, 1 August 2016 (2016-08-01), pages 941-950, XP055435179, GB ISSN: 0143-3334, DOI: 10.1093/carcin/bgw078**
- **OZAWA TSUYOSHI ET AL: "Plasma Levels of Pirnas as Biomarkers for Prognosis and Predicting Tumor Recurrence in Colorectal Cancer Patients", GASTROENTEROLOGY, vol. 152, no. 5, 30 April 2017 (2017-04-30), XP029979443, ISSN: 0016-5085, DOI: 10.1016/S0016-5085(17)30832-6**
- **Karolína Stítkovcová: "Masarykova univerzita", , 31 December 2016 (2016-12-31), pages 1-74, XP055435213, Retrieved from the Internet: URL:https://is.muni.cz/th/322481/prif_b/K. Stitkovcova-Soubor_pristupny_verejnosti.pd f [retrieved on 2017-12-14]**
- **JIE HIN: "SA1979: The Role of piRNA-823 in Human Colorectal Cancer: From Deep Sequencing Analysis to Functional Validation", GASTROENTEROLOGY, vol. 148, no. 4 Supp 1, 1 April 2015 (2015-04-01), page s373, XP055435219, DOI: http://dx.doi.org/10.1016/S0016-5085(15)31 252-X**
- **Silvia Rybecká: "Studium RNA interagujících s PIWI proteiny u renálního karcinomu", , 31 December 2016 (2016-12-31), pages 1-100, XP055435233, Retrieved from the Internet: URL:https://is.muni.cz/th/375872/prif_m/St udium_RNA_interagujicich_s_PIWI_proteiny_u _renalniho_karcinomu_Silvia_Rybecka_final. pdf [retrieved on 2017-12-14]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Field of Art

The present invention relates to a method of diagnosis of colorectal cancer from body fluid samples, using piRNAs and miRNAs as biomarkers.

Background Art

Colorectal cancer (CRC) accounts for 9.7 % of all cancers, it is the third most common cancer worldwide and the fourth most common cause of cancer related deaths, accounting for more than 13% of all cancer deaths and representing the second leading cause of malignant mortality in industrialized countries. The survival rate is significantly better for early diagnosed patients. The chance of surviving CRC is closely related to the stage of the disease at diagnosis; the earlier the diagnosis, the greater the likelihood of survival. For example, there is less than a 20% chance of 5-year survival when diagnosed late in the disease timeframe (stage IV), while there is greater than 90% chance of 5-year survival when diagnosed early (stage I). The early diagnosis usually leads to possibility of curative surgical intervention which is feasible only for patients with disease limited to primary tumor and regional lymph nodes.
Most colorectal cancers develop slowly, beginning as small benign colorectal adenomas which progress over several decades to larger and more dysplastic lesions which eventually become malignant. This gradual progression provides multiple opportunities for prevention and intervention. As early diagnosis offers the best chance for a curative treatment, CRC patients would greatly benefit from early detection because of the effectiveness of surgical treatment early on.
Unfortunately, most cases of CRC are still diagnosed in the already advanced stages when a curative surgical treatment is not possible, and chemotherapy remains the only option in spite of high costs and undesirable side effects. Because of the absence of efficient diagnostic methods of CRC, patients with colorectal neoplasia would most benefit from efficient early diagnostic CRC tests that can reveal early stages of CRC, thereby enabling preventive interventions.
The risk of CRC begins to increase after the age of 50; thereafter the risk continues to rise, approximately doubling with each succeeding decade. Increased risk is slower in women and, before age 75, women have a lower incidence of CRC than men.
The most common and earliest detection procedures available at present for CRC are (i) the fecal occult blood test (FOBT), which is based on the assumption that cancers will bleed, and can therefore be detected in the stool using chemical or immunological assays; significant tumor size must typically exist before fecal blood is detected; (ii) imaging methods such as virtual colonoscopy; and (iii) invasive methods that identify gross abnormalities such as sigmoidoscopy or colonoscopy.
The FOBT is the most widespread clinically useful test used for CRC, and involves a crude test for the peroxidase-like activity of heme in hemoglobin (guayac test) or a specific antibody against hemoglobin (Faecal Immunochemical testing, FIT). However, this screening technique suffers from a certain number of handicaps: The major drawback is its mediocre sensitivity of approximately 50%, with 20% sensitivity for adenomas (which, if they are large in size, will result in the development of cancer in 1 case out of 10), due to the fact that not all adenomas and CRCs bleed. The test is also not very specific as the appearance of blood in the stools may be related to a non-tumour condition (e.g. ulcerative colitis, hemorrhoids, fistulae, etc.). Thus, a colonoscopy must also be carried out, with the drawbacks described below.
Computerized Tomography Colonography (CTC), or virtual colonoscopy, is a recent non-invasive technique for imaging the colon, with reports varying dramatically on the performance characteristics of the assay (ranging between 39% and 94% specificity), due primarily to technological differences in the patient preparation and the hardware and software used for the analysis. Other limitations of CTC include high false-positive readings, inability to detect flat adenomas, no capacity to remove polyps, repetitive and cumulative radiation doses, and cost.
Invasive methods for identifying gross abnormalities include sigmoidoscopy and colonoscopy. Colonoscopy is usually the preferred method for screening average and increased-risk individuals over the age of 50 who have a history of CRC or prior adenomatous polyps, or other predisposing diseases such as inflammatory bowel disease. Although colonoscopy is still the standard test for the presence or absence of polyps and CRC, it can miss up to 15% of lesions >1 cm in diameter. Complications with colonoscopy can include perforation, hemorrhage, respiratory depression, arrhythmias, and infection. Approximately one in 1,000 patients suffers perforations and three in 1,000 experience hemorrhaging. Between one and three deaths out of 10,000 tests occur as a result of the procedure. Other disadvantages such as the lack of trained personnel, patient discomfort, and high cost and the low level of compliancy (around 2%) for colonoscopy will likely prevent the colonoscopy from becoming a routine CRC screening method for the general population. Most sporadic CRCs are thought to develop from benign adenomas, of which only a small number will ever develop to malignancy. Given that the time period for malignant development from benign adenoma is five to ten years, the detection of adenomas across the general population by colonoscopy would require a gross overtreatment of patients, being both costly and potentially harmful. Colonoscopy is the next test after a positive FOBT, and with a 60% false positive rate, imposes unnecessary risks that require improvement.

Therefore, biomarkers enabling early diagnosis, preferably also prognosis, and later on accurate monitoring of patients' progression and response to treatment are highly needed. Biomarkers present in samples that can be obtained from the patients by low-invasive or non-invasive methods (such as blood samples) are preferred, as they can be used in population screening.

Currently, there is no serological screening test or specific diagnostic test which detects early CRC. Warren et al (BMC Med 9 (2011) 133) have recently described Septin 9 methylation as a sensitive and specific blood test for colorectal cancer. Although there is a commercial test it is not routinely used for screening, since this test is labour intensive and expensive.

In the recent years a large amount of so-called CRC specific genes has been reported. The vast majority of the corresponding research papers or patent applications are based on data obtained by analysis of RNA expression patterns in colon cancer tissue versus a different tissue, an adjacent normal tissue or healthy colon tissue. Such approaches may be summarized as differential mRNA display techniques. However, differences on the level of mRNA are not necessarily mirrored by the level of the corresponding proteins. A protein encoded by a rare mRNA may be found in very high amounts and a protein encoded by an abundant mRNA may nonetheless be hard to detect and find at all. This lack of correlation between mRNA-level and protein level is due to reasons like mRNA stability, efficiency of translation, stability of the protein, etc.

There also are recent approaches investigating the differences in protein patterns between different tissues or between healthy and diseased tissue in order to identify candidate marker molecules which might be used in the diagnosis of CRC. Bruenagel et al. (Cancer Research 62 (2002) 2437-2442), have identified seven nuclear matrix proteins which appear to be more abundant in CRC tissue as compared to adjacent normal tissue. Further, WO 2010/061996 A1 discloses several protein biomarkers, some which can be detected in serum of CRC patients.

Other representative examples known as molecular markers of colorectal cancer are as follows: WO 2005/015224 discloses a method to diagnose colorectal cancer using an antibody against protein RLA-0 (60S acidic ribosomal protein P0). WO 2004/079368 discloses that HSP90 is highly expressed in colorectal cancers. WO 2004/071267 describes a method for diagnosing colorectal cancer in an early stage by measuring NNMT (nicotinamide N-methyltransferase) in a stool sample, and WO 2005/015234 discloses that SAHH (S-adenosylhomocysteine hydrolase) protein is able to be utilized in the diagnosis of colorectal cancer. In addition, U.S. Pat. No. 7,501,243 discloses TTK (Tyrosine threonine kinase) as a colon cancer marker.

However, despite candidate protein markers being proposed, to date their clinical/diagnostic utility has not been demonstrated. Ideally, a new diagnostic marker as a single marker should also lead to a progress in diagnostic sensitivity and/or specificity either if used alone or in combination with one or more other markers, compared to currently used diagnostic methods. At present, for example, diagnostic blood tests based on the detection of carcinoembryonic antigen (CEA) or CA19-9 are available to assist diagnosis in the field of CRC. The development of the first assays of serum carcinoembryonic antigen (CEA) in 1965 raised considerable hope on the possibility of screening test in blood. However, subsequent work showed that the sensitivity of CEA was less than 35% in individuals with invasive cancer; it could not detect early forms and its specificity was insufficient, since its serum levels increase in several pathologies. The limitations of the CA19-9 antigen, introduced more recently, are comparable to those of CEA. The current use of these two molecules is limited to post-therapeutic surveillance. Moreover, these biomarker is expressed by other types of cancer, and in benign pathologies. Despite everything, it is possible to increase sensitivity without losing specificity by combining, with CEA, another tumor marker.

Recently, signature panels of circulating miRNAs allowing a non-invasive early detection and prognosis prediction of colon cancer from a body fluid (in particular, blood serum) of the patients were reported in Vychytilova-Faltejskova et al., Carcinogenesis, 2016, 941-950. The diagnostic panel comprised miR-23a-3p, miR-27a-3p, miR-142-5p, miR-276c-3p and showed sensitivity of 89 % and specificity of 81 % in the independent validation. The prognostic panel comprised miR-23a-3p, miR-276c-3p. However, some members of this diagnostic and prognostic panel were described as being involved with other solid cancers than colorectal cancer, and thus their specificity in population screening might be compromised.

Taken together, above mentioned diagnostic approaches suffer from low sensitivity in detecting early stages (I and II) of colorectal cancer and no definite biomarkers have been identified to date that can be reliably used for detecting CRC in blood samples. Thus, there is a clinical need for identifying specific biomarkers for early detection of CRC that can be tested in a noninvasive manner.

The present invention aims at identifying body fluid biomarkers suitable for diagnosing and prognosing CRC which could be used in screening tests and would be relevant also in early stages of the disease, to increase the survival rates. The desirable tests would comprise as many as possible of the following features: average-risk, asymptomatic individuals, highly sensitive, non-invasive, low-risk, cost- effective, and ease of implementation across a large population.

Disclosure of the Invention

The present invention provides newly identified piRNAs which are useful as colorectal cancer biomarkers. piRNAs (or PIWI-interacting RNAs) are small non-coding RNA molecules expressed in eukaryotic cells. They are distinct from miRNAs in size (26-31 nucleotides rather than 21-24 nucleotides), lack of sequence conservation and increased complexity. They seem to be more specific markers than miRNAs Ozawa et al., Gastroenterology, 2017:152(5) suppl. 1, page S-152 describe that plasma levels of piRNAs can be used as biomarkers for prognosis and predicting tumor recurrence in colorectal cancer patients.

The newly identified piRNAs are piRNA-hsa-5937 (or piR-5937, alias piR-43771), having the sequence TCCCTGGTGGTCTAGTGGTTAGGATTCGGCA (SEQ ID NO. 1), and piR-28876, having the sequence GTTTCCGTAGTGTAGTGGTCATCACGTTCGC (SEQ ID NO. 15). Both piRNAs are useful as colorectal cancer biomarkers which are downregulated in cancer patients.

Furthermore, said piRNAs may be used in biomarker combination with other piRNAs and miRNAs. The expression levels of circulating piRNAs and miRNAs are used, and the diagnostic and/or prognostic methods are carried out using samples of body fluids from patients. The body fluids that can be used include in particular blood, blood serum, blood plasma, urine and saliva.

Therefore, in a first aspect of the present invention, a method for diagnosing colorectal cancer is provided, wherein the level of expression of piRNA-hsa-5937 is determined in a sample of body fluid, then the level of expression of piRNA-hsa-5937 in the sample is compared with a level of expression of piRNA-hsa-5937 in the body fluid of a healthy human, whereas when the level of expression of piRNA-hsa-5937 in the sample is lower than the level of expression of piRNA-hsa-5937 in the body fluid of a healthy human, colorectal cancer is diagnosed in the sample. The piRNA-hsa-5937 is used in this invention together with miR-23a-3p (as described herein below) and optionally also together with additional miRNAs.

The level of expression in body fluid of a healthy human is obtained by establishing the level of expression in body fluid samples obtained from healthy volunteers not suffering from colorectal cancer. The average level of expression can be obtained by known statistical methods if desired.

In order to compare the levels of expression, it is advantageous when the content of piRNA-hsa-5937 in the sample is referenced to a substance having a constant content in body fluid (i.e., normalized). Such substance may be miR-93-5p, having the sequence CAAAGUGCUGUUCGUGCAGGUAG (SEQ ID NO. 2).

The establishing of the outcome of the method might be based on the following: If the normalized level is lower than a pre-determined cut-off value then the sample is positive (colorectal cancer is diagnosed). If the normalized level is higher than a pre-determined cut-off value then the sample is negative (colorectal cancer is not diagnosed). The predetermined cut-off value can be obtained and determined using known methodologies, e.g. a statistical analysis of the normalized body fluid levels in groups of healthy volunteers (controls) and colorectal cancer patients.

In one embodiment, normalized blood serum levels of piRNA-hsa-5937 in a sample are $X = 2^{-(Ct.piR-Ct.miR93)}$. If the normalized level is lower than a pre-determined cut-off value then the sample is positive (colorectal cancer is diagnosed). If the normalized level is higher than a pre-determined cut-off value then the sample is negative (colorectal cancer is not diagnosed).

The diagnostic method based on determination of the level of expression of piRNA-hsa-5937 in body fluid sample may achieve a sensitivity of 92.5 % and specificity of 82.5 %.

In a second aspect of the invention, a method of prognosis of overall survival of colorectal cancer patient is provided, wherein the level of expression of piRNA-hsa-5937 is determined in a sample of body fluid, then the level of expression of piRNA-hsa-5937 is compared with a reference expression level, whereas the level of expression of piRNA-hsa-5937 in the sample which is higher than the reference expression level indicates a favourable overall survival prognosis, while the level of expression of piRNA-hsa-5937 in the sample which is lower than the reference expression level indicates a poor overall survival prognosis.

The reference expression level is obtained from a statistical analysis of samples from a group of colorectal cancer patients with favourable overall survival (for example at least more than 3 years from diagnosis) time and a group of colorectal cancer patients with poor overall survival time (for example less than 3 years from diagnosis). The methods of obtaining and assessing the data, and of performing the statistical analysis are known to the skilled person. For example, Receiver Operating Characteristics (ROC) analysis method may be used for identifying the reference expression level.

The favourable overall survival prognosis means a prognosis of overall survival of at least a predetermined number of years or months, for example a prognosis of overall survival of at least 20 or 24 or 26 months or 3 years from diagnosis.

The poor overall survival prognosis means a prognosis of overall survival of less than a predetermined number of years or months, for example a prognosis of overall survival of less than 20 or 24 or 26 months or 3 years from diagnosis.

In order to compare the levels of expression, it is advantageous when the content of piRNA-hsa-5937 in the sample is referenced to a substance having a constant content in body fluid, such as miR-93-5p.

The method for diagnosing colorectal cancer using piRNA-hsa-5937 as a marker further includes determining the levels of expression in the body fluid sample of
miR-23a-3p, having the sequence AUCACAUUGCCAGGGAUUUCC (SEQ ID NO. 3),
and optionally also of at least one miRNA selected from:

miR-23a-3p, having the sequence AUCACAUUGCCAGGGAUUUCC (SEQ ID NO. 3),
miR-27a-3p, having the sequence UUCACAGUGGCUAAGUUCCGC (SEQ ID NO. 4),
miR-142-5p, having the sequence CAUAAAGUAGAAAGCACUACU (SEQ ID NO. 5),

comparing them with the levels of expression of the same miRNAs in body fluid of a healthy human, whereas when the the level of expression of said miRNAs in the sample is higher than the level of expression of said miRNAs in body fluid of a healthy human, colorectal cancer is diagnosed in the sample.

In order to compare the levels of expression, it is advantageous when the content of the above-defined miRNAs in the sample is referenced to a substance having a constant content in body fluid (i.e., a normalizing marker). Preferably, the normalizing marker is miR-93-5p.

In one embodiment, diagnostic score (DxScore) can be calculated from the levels of expression, or preferably from the normalized levels of expression of the biomarkers. Value of the diagnostic score can then be used for classifying the patients into patients suffering from colorectal cancer or patients not suffering from colorectal cancer.

If the DxScore is higher than a cut-off value, the sample is positive (colorectal cancer is diagnosed), and if the DxScore is lower than a cut-off value, the sample is negative (colorectal cancer is not diagnosed). The cut-off value can be obtained using known methodologies, e.g. a statistical analysis of the normalized body fluid levels in groups of healthy volunteers (controls) and colorectal cancer patients.

Using the DxScore, the method allows to achieve the sensitivity of 96.25 % and specificity of 97.50 %.

In one embodiment, normalized serum level of hsa-miR-23a in the sample is calculated as X(miR-23a) = $2^{-(Ct.miR23a - Ct.miR93)}$, normalized serum level of hsa-miR-27a in the sample is calculated as X(miR-27a) = $2^{-(Ct\text{-}miR27a - Ct.miR93)}$, normalized serum level of hsa-miR-142-5p in the sample is calculated as X(miR-142-5p) = $2^{-(Ct.miR142\text{-}5p - Ct.miR93)}$, normalized serum level of piRNA-hsa-5937 in the sample is calculated as X(piR-5937) = $2^{-(Ct.piR5937 - Ct.miR93)}$.

The following formula can then be used for calculating the diagnostic score (DxScore):

$$DxScore = -0{,}8895 + 62{,}92648 * X(miR\text{-}23a) - 8{,}08928 * X(miR\text{-}27a) + 73{,}16745 * X(miR\text{-}142\text{-}5p) - 1{,}6757 * X(piR\text{-}5937).$$

The normalized levels of the biomarkers and diagnostic score (DxScore) may be used, Preferably, the normalizing marker is miR-93-5p. If the normalized levels or the DxScore are lower than a pre-determined cut-off value then the sample is positive (colorectal cancer is diagnosed). If the normalized level levels or the DxScore are higher than a pre-determined cut-off value then the sample is negative (colorectal cancer is not diagnosed). The pre-determined cut-off value and/or the DxScore can be obtained and determined using known methodologies, e.g. a statistical analysis of the normalized body fluid levels in groups of healthy volunteers (controls) and colorectal cancer patients.

Yet further, the method for diagnosing colorectal cancer may further include determining of levels of expression in the body fluid sample of at least one of the following piRNAs, comparing them with the levels of expression of the same piRNA, respectively, in body fluid of a healthy human, whereas when the level of expression of said piRNA shows the change determining colorectal cancer relative to the level of expression of the same piRNA in body fluid of a healthy human, colorectal cancer is diagnosed in the sample:

| piRNA | Sequence | Change in expression level determining colorectal cancer |
|---|---|---|
| piR-hsa-32161 | CCCCCCACTGCTAAATTTGACTGG (SEQ ID NO. 6) | decrease |
| piR-hsa-32238 | CCCCCACTGCTAAATTTGACTGGTT (SEQ ID NO. 7) | decrease |

(continued)

| piRNA | Sequence | Change in expression level determining colorectal cancer |
|---|---|---|
| piR-hsa-1242 | AGCCCGGCTAGCTCAGTCGGTAGAGCATGAG (SEQ ID NO. 8) | decrease |
| piR-hsa-32187 | CCCCCCACTGCTAAATTTGACTG (SEQ ID NO. 9) | decrease |
| piR-hsa-27620 | GCCCGGCTAGCTCAGTCGGTAGAGCATGAG (SEQ ID NO. 10) | decrease |
| piR-hsa-32162 | CCCCCCACTGCTAAATTTGACTGGC (SEQ ID NO. 11) | decrease |
| piR-hsa-32195 | CCCCCCACTGCTAAATTTGACTGGT (SEQ ID NO. 12) | decrease |
| piR-hsa-32158 | CCCCCCACTGCTAAATTTGACTGGT (SEQ ID NO. 13) | decrease |
| piR-hsa-29218 | TAAAGTGCTGACAGTGCAGATAGTGGTCCTC (SEQ ID NO. 14) | decrease |
| piR-hsa-28876 | GTTTCCGTAGTGTAGTGGTCATCACGTTCGC (SEQ ID NO. 15) | decrease |
| piR-hsa-24672 | TTCCCTGGTGGTCTAGTGGTTAGGATTCGGC (SEQ ID NO. 16) | decrease |
| piR-hsa-26872 | GAGGAATGATGACAAGAAAAGGCCGAA (SEQ ID NO. 17) | increase |
| piR-hsa-8226 | TCTGCTGCCTCAGCCTCCCGAGTAGCTGA (SEQ ID NO. 18) | increase |
| piR-hsa-30715 | TACTCAGGAGGCTGAGGCAGGAGAATGGC (SEQ ID NO. 19) | increase |
| piR-hsa-6746 | TCCTGGGTTCAGGTGATTCTCCTGCCTCAGT (SEQ ID NO. 20) | increase |
| piR-hsa-30937 | TAGTCCCAGCTACTTGGGAGGCTGAGGCA (SEQ ID NO. 21) | increase |
| piR-hsa-26543 | GACCAGCCTGGCCAACA TGGTGAAACCCCGCC(SEQ ID NO.22) | increase |

(continued)

| piRNA | Sequence | Change in expression level determining colorectal cancer |
|---|---|---|
| piR-hsa-15278 | TGCCTCCCGGATTCAAGTGATTCTCCTGCCT (SEQ ID NO. 23) | increase |
| piR-hsa-5505 | TCCCAGCTACCTAGGAGGCTGAGGCAGGAG (SEQ ID NO. 24) | increase |
| piR-hsa-30714 | TACTCAGGAGGCTGAGACAGGAGAATTGC (SEQ ID NO. 25) | increase |
| piR-hsa-1359 | ATCGAGGCTAGAGTCACGCTTGGGTATCGGCT (SEQ ID NO.26) | decrease |
| piR-hsa-27139 | GCCTGGGTAGCTCAGTCGGTAGAGCATCAGA (SEQ ID NO. 27) | decrease |
| piR-hsa-28488 | GGTCGCTGGTTCGAATCCGGCTCGAAGGACC (SEQ ID NO. 28) | decrease |
| piR-hsa-27616 | GCCCGGATGATCCTCAGTGGTCTGGGGTGC (SEQ ID NO. 29) | decrease |
| piR-hsa-29716 | TAAGGTGCATCTAGTGCAGATAGTGAAGTA (SEQ ID NO. 30) | decrease |
| piR-hsa-28019 | GGAGGTGATGAACTGTCTGAGCCTGACC (SEQ ID NO. 31) | decrease |
| piR-hsa-23231 | CCCCTGGTGGTCTAGTGGTTAGGATTCGGC (SEQ ID NO. 32) | decrease |
| piR-hsa-28846 | GTTCACTGATGAGAGCATTGTTCTGAGCCA (SEQ ID NO. 33) | decrease |
| piR-hsa-24360 | TTCACTGATGAGAGCATTGTTCTGAGC (SEQ ID NO. 34) | decrease |
| piR-hsa-6046 | TCCGATCCTCGTTGTTTTGGCTATGGCCAGA (SEQ ID NO. 35) | decrease |
| piR-hsa-23940 | CTGACCTCAAGTGATCCACCTGCCTCAGCC (SEQ ID NO. 36) | increase |
| piR-hsa-32182 | CCCCCACTGCTAAATTTGACTGGCTA (SEQ ID NO. 37) | decrease |

(continued)

| piRNA | Sequence | Change in expression level determining colorectal cancer |
|---|---|---|
| piR-hsa-32167 | CCCCCACTGCTAAATTTGACTGGCT (SEQ ID NO. 38) | decrease |
| piR-hsa-28190 | GGCCGTGATCGTATAGTGGTTAGTACTCTG (SEQ ID NO. 39) | decrease |
| piR-hsa-23210 | CCCCCCACTGCTAAATTTGACTGGTT (SEQ ID NO. 40) | decrease |
| piR-hsa-11362 | TGAGGTAGTAGGTTGTATGGTTTAGAGTTACA (SEQ ID NO.41) | decrease |
| piR-hsa-23209 | CCCCCCACTGCTAAATTTGACTGGCTA (SEQ ID NO. 42) | decrease |
| piR-hsa-32159 | CCCCCCACTGCTAAATTTGACTGGCT (SEQ ID NO. 43) | decrease |

All miRNAs and piRNAs used in the present invention can be referenced to the same substance having a constant content in body fluid, such as miR-93-5p.

In one embodiment, diagnostic score (DxScore) can be calculated from the levels of expression, or preferably from the normalized levels of expression of the biomarkers. Value of the diagnostic score can then be used for classifying the patients into patients suffering from colorectal cancer or patients not suffering from colorectal cancer.

The pre-determined cut-off value and/or the DxScore can be obtained and determined using known methodologies, e.g. a statistical analysis of the normalized body fluid levels in groups of healthy volunteers (controls) and colorectal cancer patients.

The present diagnostic method allows to diagnose colorectal cancer even in a very early stage (I and II clinical stage), which strongly increases the chances of successful treatment for the patients. It may be carried out with only one piRNA, and its sensitivity and specificity may be further improved by addition of further markers. The sensitivities and specificities achieved for early stage samples indicate that the present diagnostic method is very suitable for use in screening of population. In contrast to diagnostic methods used currently in the clinical practice, the method of the present invention is non-invasive, increases the patient comfort, and does not require complex equipment for taking the sample from the patient. These benefits will result in more people willing to participate in the screening, and in earlier detection of the colorectal cancer which greatly improves the chances of recovery and complete healing of the patients.

The CRC-specific biomarkers of the invention are circulating piRNAs and miRNAs, i.e., their levels in body fluids are indicative of diagnosis and prognosis of CRC. A test based on these biomarkers can be widely accepted by the general population as it is minimally invasive and can be used to monitor an individual's susceptibility to disease prior to resorting to, or in combination with, conventional screening methods. This will be extremely beneficial in the management of CRC risk, prevention, and treatment.

From this perspective, detection of small non-coding RNAs (microRNAs and PIWI-interacting RNAs) presents a very promising diagnostic approach.

MicroRNAs (miRNAs) are important regulators of gene expression comprising an abundant class of endogenous, small noncoding RNAs (18-25 nucleotides in length). They are capable of either promoting mRNA degradation or attenuating protein translation. Bioinformatic studies have estimated that miRNAs may regulate more than 50 % of all human genes and each miRNA can control hundreds of gene targets. Some miRNAs are expressed in a cell-specific, tissue-specific and/or developmental stage-specific manner, while others are expressed ubiquitously. The number of verified miRNAs is still growing - the latest version of web-based database miRBase has annotated over 1800 precursor and 2342 mature sequences in the human genome. Based on the annotations for the genomic position of miRNAs which indicated that a vast majority of miRNAs is located in intergenic regions (>1 kb away from annotated or predicted genes), it has been postulated that most miRNA genes are transcribed as autonomous transcription units.

MiRNAs may serve as master regulators of many fundamental biological processes, such as embryogenesis, organ development, cellular differentiation, proliferation, apoptosis, etc., affecting such major biological systems as sternness and immunity.

When compared to mRNAs, microRNAs represent far more suitable biomarkers due to their long half-life, high stability under conditions that include RNase exposure, extremes of pH, long-term storage, and multiple freeze-thaw cycles.

Piwi-interacting RNA (piRNA) is the largest class of small non-coding RNA molecules expressed in animal cells. piRNAs form RNA-protein complexes through interactions with piwi proteins. They are distinct from microRNA (miRNA) in size (26-31 nt rather than 21-24 nt), lack of sequence conservation, and increased complexity. These small RNAs regulate gene expression on transcriptional and post-transcriptional level, however, they are preferentially involved in silencing of transponable elements LINE and SINE and thus contributing to genomic stability. Further, piRNAs participate also in other important biological processes, such as gametogenesis, chromosome segregation, or stem cell self-renewal. Although their expression has been firstly described in germ line cells, it has been proved that they are present in all tissue types and their expression is highly tissue-specific. In addition, the deregulated expression of piRNAs in tumor tissues has been described. Nevertheless, the exact function of these molecules in cancerogenesis is not known. Recently, free circulating piRNAs have been reported to be stably present in body fluids.

Detection of biomarkers:

The detection of piRNA and miRNA biomarkers of the present invention may be carried out by known methods, such as a real-time PCR assay, a micro-array assay, histochemistry assay, an immunological assay or sequencing assay.

Both miRNAs and piRNAs can be detected by standard real-time RT (Reverse-transcription) PCR technologies available at hospital-level laboratory medicine departments. The main advantages of real-time RT-PCR in comparison to serological tests is its high sensitivity, reliability and specificity. These days the costs of individual PCR miRNA/piRNA assays is low enough to enable its population-wide application in the screening programs. Commercial assays are available to detect miRNAs/piRNAs by real-time RT-PCR (e.g. Thermo Fischer Scientific).

In RT-PCT methods, reverse transcription (RT) is first utilized to convert the target RNA into its cDNA, which is then subsequently amplified and quantified via one of several conventional PCR methods. However, the simple translation of these methods to miRNAs/piRNAs is complicated by the short size of the target, as the length of the primers normally used in the PCR step are as long as mature miRNAs/piRNAs themselves. Shorter primers are typically not useful, as their low duplex melting temperature with the miRNA/piRNAs can introduce signal bias. To avoid these challenges, researchers have developed several of creative approaches based upon the enzymatic modification of conventional primers or altogether new primers for mature miRNAs/piRNAs. Currently, there are two main methods for quantitative PCR of miRNAs/piRNAs (stem-loop RT-based method, and polydenylation based methods).

Statistical methods:

The receiver operating characteristic (ROC) curve is the standard analytical tool for evaluating diagnostic tests. The diagnostic accuracy of a biomarker is most commonly measured by calculating its sensitivity and specificity. Sensitivity is the proportion of patients who are correctly categorized as having disease among those who truly have the disease. Similarly, specificity is the proportion of patients who are correctly categorized as not having the disease among all patients who truly don't have the disease. Since most diagnostic biomarkers provide results in the continuous scale, the sensitivity and specificity of the test depends on the specific threshold selected. ROC analysis is a statistical method to identify these specific thresholds. The area under the ROC curve (AUC) is the average sensitivity of the biomarker over the range of specificities. It is often used as a summary statistic representing the overall performance of the biomarker. A biomarker with no predictive value would have an AUC of 0.5 (also represented by the diagonal "chance" line above), while a biomarker with perfect ability to predict disease would have an AUC of 1.

Brief Description of Drawings

Figure 1 Validation of blood serum piR-5937 (A,B) and piR-28876 (C,D) as diagnostic biomarkers in colorectal cancer. A,C-Mann-Whitney test, B,D - ROC analysis.

Figure 2 Blood serum levels of piR-5937 are strongly correlated with overall survival of colorectal cancer patients (longrank test, $P<0.0001$).

Figure 3 MiRNAs and piRNA levels in colorectal cancer patients (all clinical stages, TNM I-IV) and healthy controls (***, $P<0.0001$).

Figure 4 MiRNAs and piRNA levels in colorectal cancer patients (early stages- TNM I a II) and healthy controls.
Figure 5 Validation of DxScore1 based on miR-23a-3p, miR-27a-3p, miR-142-5p and piR-5937 serum levels in all stages colorectal cancer patients (A,C) and early stages colorectal cancer patients (B,D).
Figure 6 Validation of DxScore2 based on miR-23a-3p and piR-5937 serum levels in all stages colorectal cancer patients (A,C) and early stages colorectal cancer patients (B,D).

Examples

**Patients and methods**

Patients

Blood serum samples were collected from 144 colorectal cancer cases (36 patients in TNM stage I, 36 patients in TNM stage II, 36 patients in TNM stage III, 36 patients in TNM stage IV; 82 men, 62 women; mean age 65 years) and 96 controls (48 men, 48 women; mean age 62 years) included in the piRNA profiling study. For the independent validation of candidate piRNAs, another 80 cases (19 patients in TNM stage I, 21 patients in TNM stage II, 21 patients in TNM stage III, 19 patients in TNM stage IV; 44 men, 36 women; mean age 65 years) and 80 controls (47 men, 33 women; mean age 60 years) were included. In addition, another 90 colorectal cancer cases with early stage of the disease (40 patients in TNM stage 1 and 50 patients in TNM stage 2, 50 men, 40 women; mean age 61 years) and 100 controls (52 men, 48 women; mean age 59 years) were used for evaluation of identified miRNA/piRNA biomarkers and models. All subjects enrolled in the study were of the same ethnicity (European descent), and colorectal cancer patients did not receive any neo-adjuvant treatment. Hemolytic serum samples were excluded from the study, as hemolysis may influence the expression of some miRNAs. Written informed consent was obtained from all participants (patients, healthy donors), and the study was approved by the local Ethics Board at Masaryk Memorial Cancer Institute.

RNA extraction

Before RNA extraction, all samples were checked for hemolysis using the Harboe's spectrophotometric method, which utilizes a 3 point (380 nm, 415 nm, and 450 nm) Allen correction method to quantify hemoglobin concentration. Only the samples with hemoglobin concentration lower than 5 mg•dl-1 were further used in this study. Total RNA enriched for small RNAs was isolated from blood serum using Qiagen miRNeasy Serum/Plasma Kit (Qiagen, GmbH, Hilden, Germany) according to the modified manufacturers' protocol. Briefly, 250 μl of serum was thawed on ice and centrifuged at 14000 × g at 4 °C for 5 minutes to remove cellular debris. Subsequently, 200 μl of supernatant was lysed with 1 ml of QIAzol Lysis Reagent. For each sample, 1.25 μl of 0.8 μg·μl-1 MS2 RNA carrier (cat. no. 10165948001, Roche, Basel, Switzerland) was added to 1 ml of QIAzol solution. The extracted RNA was eluted by 2 × 20 μl of preheated Elution Solution. For the purposes of library preparation, RNA was always isolated separately from 12 samples (12 × 250 μl; in case of colon cancer patients, all 12 patients were of the same stage. After phase separation, upper aqueous phase from all 12 samples was combined, mixed with 1.5 volume of 100% ethanol and pipetted into one RNeasy MinElute spin column. Elution was performed using 14 μl of preheated Elution Solution. The concentration and purity of RNA were determined spectrophotometrically by measuring its optical density (A260/280 > 2.0; A260/230 > 1.8) using NanoDrop ND-1000 Spectrophotometer (Thermo Fisher Scientific, Wilmington, DE, USA). Further, the concentrations and quality of RNA of pooled samples for NGS were also measured using Qubit 2.0 Fluorometer (Thermo Fisher Scientific) and Agilent 2100 Bioanalyzer (Agilent Technologies, Santa Clara, CA, USA). The samples were either stored at -80 °C or further processed.

Small RNA library construction and sequencing

All libraries were prepared using the Illumina TruSeq Small RNA protocol (Illumina, San Diego, CA, USA) following the manufacturer's instructions. Briefly, at least 1 μg of total RNA enriched for small RNA was used, and a pair of adaptors was ligated to the 3' and 5' ends of piRNAs. Subsequently, 13 cycles of PCR amplification with unique barcode-labeled amplification primers were performed, and size-selection was conducted on 6% native polyacrylamide gel. Then, cDNA fragments between 145 and 160 bp corresponding to the piRNA populations were excised from the gel, eluted and precipitated using ethanol precipitation. The final cDNA pellet was air dried and resuspended in 8 μl of nuclease-free water. The concentration of prepared libraries was measured using High Sensitivity DNA chip and Agilent 2100 Bioanalyzer (Agilent Technologies, Santa Clara, CA, USA). Equimolar amounts of each library were pooled at a final concentration of 2nM cDNA, and samples were sequenced on a flowcell with 50-bp single-end reads using MiSeq sequencer (Illumina, San Diego, CA, USA).

Sequencing data processing and differential piRNA analyses

Count-based miRNA expression data were generated by the Chimira tool from fastq files. All sequences were adapter trimmed and mapped against piRBase allowing up to two mismatches per sequence. Further analyses were performed using R/Bioconductor packages. piRNAs having less than 1 read per million in more than 17 pooled samples were dropped out. The read counts were pre-normalized by adding normalization factors within edgeR package and further between-sample normalized by the voom function in LIMMA package. After the normalized expression levels were determined, the differentially expressed piRNAs between colorectal cancer patients and healthy controls were screened applying linear model fitting and a Bayes approach. The obtained P values were adjusted for multiple testing using the Benjamini-Hochberg method.

Reverse transcription and quantitative real-time PCR

Complementary DNA was synthesized from total RNA using gene-specific primers according to the TaqMan MicroRNA Assay protocol (Applied Biosystems, Foster City, CA, USA). For reverse transcriptase reactions, 10 ng of RNA sample, 50 nM of stem loop RT primer (from miR-23a-3p, miR-27a-3p, miR-142-5p, piR-5937 Applied Biosystems, Thermo Fisher, USA), 1 × RT buffer, 0.25 mM each of dNTPs, 3.33 U.μl-1 MultiScribe reverse transcriptase and 0.25 U.μl-1 RNase inhibitor (all from TaqMan MicroRNA Reverse Transcription kit, Applied Biosystems, Foster City, CA, USA) were used. Reaction mixtures (10 μl) were incubated for 30 min at 16 °C, 30 min at 42 °C, 5 min at 85 °C and then held at 4 °C (T100TM Thermal Cycler; Bio-Rad, Hercules, CA, USA). Real-time PCR was performed using the QuantStudio 12K Flex Real-Time PCR system (Applied Biosystems, Foster City, CA, USA). The 20-μl PCR reaction mixture included 1.33 μl of RT product, 1 × TaqMan (NoUmpErase UNG) Universal PCR Master Mix and 1 μl of primer (from miR-23a-3p, miR-27a-3p, miR-142-5p, piR-5937 assays) and probe mix of the TaqMan MicroRNA Assay kit (Applied Biosystems, Foster City, CA, USA). Reactions were incubated in a 96 well optical plate at 95 °C for 10 min, followed by 40 cycles at 95 °C for 15 s and 60 °C for 1 min.

Data normalization and statistical analyses

The threshold cycle data were calculated by QuantStudio 12K Flex software (Applied Biosystems, Foster City, CA, USA). All real-time PCR reactions were run in triplicates. The average expression levels of all measured miRNAs were normalized using miR-93-5p (Assay No. 001090; Applied Biosystems, Foster City, CA, USA) and subsequently analyzed by the $2^{-\Delta Ct}$ method. MiR 93-5p was selected as an endogenous control through combination of standard geNorm and NormFinder algorithms (see Vychytilova-Faltejskova et al, 2016). Statistical differences between the levels of analyzed miRNAs in serum samples of colon cancer patients and serum samples of healthy donors were evaluated by two-tailed non-parametric Mann-Whitney test. Paired samples before and after surgery were analyzed using two-tailed non-parametric Wilcoxon test for paired samples. Analysis of Kaplan-Meier survival curves and longrank test was performed to evaluate prognostic potential of piR-5937. All calculations were performed using GraphPad Prism version 5.00 (GraphPad Software, La Jolla, CA, USA) and R environment (R Development Core Team). P-values of less than 0.05 were considered statistically significant.

**Example 1**

**PIWI-interacting RNAs with differential levels in body fluid of colorectal cancer patients and healthy donors**

Small RNA sequencing of RNA purified from blood serum samples from 144 colorectal cancer patients and 96 healthy controls was carried out using MiSeq sequencer (Illumina). In total, 20 small RNA libraries were prepared and sequenced (12 libraries per colon cancer patients, 8 libraries per healthy controls, RNA samples from 12 patients/healthy donors in each library). On average, more than 96% of the reads had Q-score higher than 30, thus the obtained data were considered to be of high quality. The sequenced samples contained on average 8.925.000±2.139.597 reads and 8.219.664±1.954.177 reads passed the filter. In total, 470 and 453 piRNAs were detectable (more than 50 copies per 1 million of reads) in colorectal cancer patients and healthy donors, respectively. From these, 39 miRNAs were found to have significantly different levels (10 up-regulated, 29 downregulated; Table 1) in serum samples of colorectal cancer patients compared to healthy donors (adjusted P < 0.005).

Table 1

| Summary of 39 piRNAs with significantly different levels in blood serum of colorectal cancer patients and healthy donors (adjusted P<0.005) | | | | |
|---|---|---|---|---|
| **PiRNA** | **Sequence** | **Change with CRC** | **log FC** | **p-value** |
| piR-hsa-32161 | CCCCCCACTGCTAAATTTGACTGG (SEQ ID NO. 6) | DOWN | -1,208 | $6{,}232\cdot10^{-4}$ |
| piR-hsa-32238 | CCCCCACTGCTAAATTTGACTGGTT (SEQ ID NO. 7) | DOWN | -1,114 | $3{,}002\cdot10^{-3}$ |
| piR-hsa-1242 | AGCCCGGCTAGCTCAGTCGGTAGAGCATGAG (SEQ ID NO. 8) | DOWN | -1,203 | $4{,}31\cdot10^{-4}$ |
| piR-hsa-32187 | CCCCCCACTGCTAAATTTGACTG (SEQ ID NO. 9) | DOWN | -1,173 | $2{,}292\cdot10^{-3}$ |
| piR-hsa-27620 | GCCCGGCTAGCTCAGTCGGTAGAGCATGAG (SEQ ID NO. 10) | DOWN | -1,081 | $1{,}678\cdot10^{-3}$ |
| piR-hsa-32162 | CCCCCCACTGCTAAATTTGACTGGC (SEQ ID NO. 11) | DOWN | -1,567 | $3{,}98\cdot10^{-6}$ |
| piR-hsa-32195 | CCCCCCACTGCTAAATTTGACTGGT (SEQ ID NO. 12) | DOWN | -1,048 | $1{,}0366\cdot10^{-3}$ |
| piR-hsa-32158 | CCCCCCACTGCTAAATTTGACTGGT (SEQ ID NO. 13) | DOWN | -2,021 | $2{,}1181\cdot10^{-3}$ |
| piR-hsa-29218 | TAAAGTGCTGACAGTGCAGATAGTGGTCCTC (SEQ ID NO. 14) | DOWN | -1,093 | $1{,}1209\cdot10^{-3}$ |
| piR-hsa-28876 | GTTTCCGTAGTGTAGTGGTCATCACGTTCGC (SEQ ID NO. 15) | DOWN | -2,367 | $8{,}51\cdot10^{-9}$ |
| piR-hsa-5937 | TCCCTGGTGGTCTAGTGGTTAGGATTCGGCA (SEQ ID NO.1) | DOWN | -2,999 | $8{,}46\cdot10^{-12}$ |
| piR-hsa-24672 | TTCCCTGGTGGTCTAGTGGTTAGGATTCGGC (SEQ ID NO. 16) | DOWN | -3,130 | $4{,}03\cdot10^{-11}$ |
| piR-hsa-26872 | GAGGAATGATGACAAGAAAAGGCCGAA (SEQ ID NO. 17) | UP | 1,149 | $2{,}6204\cdot10^{-3}$ |
| piR-hsa-8226 | TCTGCTGCCTCAGCCTCCCGAGTAGCTGA (SEQ ID NO. 18) | UP | 1,910 | $7{,}435\cdot10^{-4}$ |
| piR-hsa-30715 | TACTCAGGAGGCTGAGGCAGGAGAATGGC (SEQ ID NO. 19) | UP | 1,320 | $3{,}304\cdot10^{-3}$ |
| piR-hsa-6746 | TCCTGGGTTCAGGTGATTCTCCTGCCTCAGT (SEQ ID NO. 20) | UP | 1,693 | $2{,}4257\cdot10^{-3}$ |
| piR-hsa-30937 | TAGTCCCAGCTACTTGGGAGGCTGAGGCA (SEQ ID NO. 21) | UP | 1,917 | 0,0001423 |
| piR-hsa-26543 | GACCAGCCTGGCCAACATGGTGAAACCCCGCC(SEQ ID NO.22) | UP | 1,597 | $2{,}5176\cdot10^{-3}$ |
| piR-hsa-15278 | TGCCTCCCGGATTCAAGTGATTCTCCTGCCT (SEQ ID NO. 23) | UP | 1,849 | $9{,}70\cdot10^{-5}$ |
| piR-hsa-5505 | TCCCAGCTACCTAGGAGGCTGAGGCAGGAG (SEQ ID NO. 24) | UP | 1,903 | $1{,}464\cdot10^{-4}$ |
| piR-hsa-30714 | TACTCAGGAGGCTGAGACAGGAGAATTGC (SEQ ID NO. 25) | UP | 1,574 | $9{,}617\cdot10^{-4}$ |

(continued)

| Summary of 39 piRNAs with significantly different levels in blood serum of colorectal cancer patients and healthy donors (adjusted P<0.005) | | | | |
|---|---|---|---|---|
| **PiRNA** | **Sequence** | **Change with CRC** | **log FC** | **p-value** |
| piR-hsa-1359 | ATCGAGGCTAGAGTCACGCTTGGGTATCGGCT (SEQ ID NO.26) | DOWN | -1,359 | $2,6683 \cdot 10^{-3}$ |
| piR-hsa-27139 | GCCTGGGTAGCTCAGTCGGTAGAGCATCAGA (SEQ ID NO. 27) | DOWN | -1,341 | $4,7537 \cdot 10^{-3}$ |
| piR-hsa-28488 | GGTCGCTGGTTCGAATCCGGCTCGAAGGACC (SEQ ID NO. 28) | DOWN | -1,443 | $2,1103 \cdot 10^{-3}$ |
| piR-hsa-27616 | GCCCGGATGATCCTCAGTGGTCTGGGGTGC (SEQ ID NO. 29) | DOWN | -1,277 | $4,8853 \cdot 10^{-3}$ |
| piR-hsa-29716 | TAAGGTGCATCTAGTGCAGATAGTGAAGTA (SEQ ID NO. 30) | DOWN | -1,323 | $3,3901 \cdot 10^{-3}$ |
| piR-hsa-28019 | GGAGGTGATGAACTGTCTGAGCCTGACC (SEQ ID NO. 31) | DOWN | -1,945 | $3,2583 \cdot 10^{-3}$ |
| piR-hsa-23231 | CCCCTGGTGGTCTAGTGGTTAGGATTCGGC (SEQ ID NO. 32) | DOWN | -1,855 | $9,07 \cdot 10^{-5}$ |
| piR-hsa-28846 | GTTCACTGATGAGAGCATTGTTCTGAGCCA (SEQ ID NO. 33) | DOWN | -2,050 | $2,142 \cdot 10^{-4}$ |
| piR-hsa-24360 | TTCACTGATGAGAGCATTGTTCTGAGC (SEQ ID NO. 34) | DOWN | -1,430 | $4,8228 \cdot 10^{-3}$ |
| piR-hsa-6046 | TCCGATCCTCGTTGTTTTGGCTATGGCCAGA (SEQ ID NO. 35) | DOWN | -1,396 | $1,4122 \cdot 10^{-3}$ |
| piR-hsa-23940 | CTGACCTCAAGTGATCCACCTGCCTCAGCC (SEQ ID NO. 36) | UP | 1,217 | $3,495 \cdot 10^{-3}$ |
| piR-hsa-32182 | CCCCCACTGCTAAATTTGACTGGCTA (SEQ ID NO. 37) | DOWN | -1,044 | $2,0513 \cdot 10^{-3}$ |
| piR-hsa-32167 | CCCCCACTGCTAAATTTGACTGGCT (SEQ ID NO. 38) | DOWN | -1,108 | $1,2705 \cdot 10^{-3}$ |
| piR-hsa-28190 | GGCCGTGATCGTATAGTGGTTAGTACTCTG (SEQ ID NO. 39) | DOWN | -1,277 | $7,75 \cdot 10^{-5}$ |
| piR-hsa-23210 | CCCCCCACTGCTAAATTTGACTGGTT (SEQ ID NO. 40) | DOWN | -1,418 | $3,86 \cdot 10^{-5}$ |
| piR-hsa-11362 | TGAGGTAGTAGGTTGTATGGTTTAGAGTTACA (SEQ ID NO.41) | DOWN | -0,890 | $3,728 \cdot 10^{-3}$ |
| piR-hsa-23209 | CCCCCCACTGCTAAATTTGACTGGCTA (SEQ ID NO. 42) | DOWN | -1,642 | $3,79 \cdot 10^{-5}$ |
| piR-hsa-32159 | CCCCCCACTGCTAAATTTGACTGGCT (SEQ ID NO. 43) | DOWN | -1,723 | $3,02 \cdot 10^{-5}$ |

**Validation of candidate piRNAs in independent cohorts of patients and controls**

In total, serum samples from 80 colorectal cancer patients and 80 healthy donors were included in the validation phase of the study. Out of 39 piRNAs identified by smallRNA sequencing to have significantly lower levels in blood serum of colorectal cancer patients in comparison to healthy controls, piR-5937 and piR-28876 were selected as the most promising for independent validation. Both piRNAs were proved to be significantly decreased in blood serum of colorectal cancer patients compared to healthy donors: piR-5937 (P<0.0001, AUC=0.916, sensitivity = 92,5%, specificity = 82,5%), piR-28876 (P<0.0001, AUC=0.896, sensitivity = 92,5%, specificity = 72,5%) (see Figure 1). Moreover, higher levels of blood serum piR-5937 were significantly associated with more favourable survival than those with lower levels (P<0.0001; HR 0.09, CI 0.02-0.036, Figure 2), which shows its prognostic effect.

**Example 2 Development and validation of colorectal cancer diagnostic models (DxScores) based on combination of blood serum piRNAs and miRNAs levels**

The development of colorectal cancer diagnostic models (DxScores) based on piRNA/miRNA levels in blood serum was performed using logistic regression (for diagnosis). To generate diagnosis-related signature, piR-5937, piR-28876 and the top 4 miRNAs identified in the study of Vychytilova-Faltejskova et al. (2016, cited in the Background Art chapter) (miR-23a-3p, miR-27a-3p, miR-142-5p, miR-376c-3p) were introduced into a bidirectional stepwise logistic regression model on the training set of samples. The final model was taken as that which maximizes the Akaike information criterion. A ROC (receiver operating characteristic) curve was generated for further analysis of the DXscores. The maximum Youden index was used to obtain optimal cut-off value for discrimination of healthy donors and colon cancer patients. Subsequently, ROC curve analysis for each set of samples was applied to calculate the sensitivity and specificity of DXscore. Validation of the obtained model and cut-off was performed on the independent validation set of samples with early stages of the disease. This approach led to establishment of the two efficient diagnostic models: (i) based on the expression of 4 biomarkers (miR-23a-3p, miR-27a-3p, miR-142-5p and piR-5937) (DxScore1) and (ii) based on the expression of 2 biomarkers (miR-23a-3p and piR-5937) (DxScore2). Both were characterized by very high sensitivity and specificity in diagnosis of colorectal cancer from blood serum specimen. All miRNAs/piRNA included in the DxScores indicated significantly different levels in blood serum of colorectal cancer patients and healthy donors. Levels of miRNAs were higher and levels of piRNA were lower in blood serum of patients in both validation cohorts ($1^{st}$ cohort -all TNM stages, $2^{nd}$ cohort - early TNM stages) (Figures 3 and 4).

The best analytical performance indicated a model based on 4 biomarkers (miR-23a-3p, miR-27a-3p, miR-142-5p and piR-5937) (DxScore1). Diagnostic score was calculated according to the following formula :

$$\text{DxScore1} = -0{,}8895 + 62{,}92648*\text{miR-23a} - 8{,}08928*\text{miR-27a} + 73{,}16745*\text{miR-142-5p} - 1{,}6757*\text{piR-5937}$$

DxScore1 enabled to identify colorectal cancer with sensitivity 99% and specificity 90% in cohort of patients with all TNM stages (Figure 5A,C). If the same DxScore1 and cut-off value was applied in independent validation cohort of patients with early TNM stages of the disease, sensitivity was 84% and specificity 83% (Figure 5B,D), which significantly outperforms current diagnostic approaches used in colorectal cancer (summarized in Table 2).

Table 2 Summary of analytical performance of DxScore1 in diagnosis of colorectal cancer.

| | **Validation phase (TNM stage I-IV)** | **Validation phase I + II (TNM stage I + II)** |
|---|---|---|
| | | |
| **AUC** | 0.9908 | 0.9192 |
| **sensitivity** | 96.25% | 84.27% |
| **specificity** | 97.50% | 82.65% |
| **accuracy** | 96.86% | 83.46% |
| **PPV** | 90.70% | 81.72% |
| **NPV** | 97.30% | 85.26% |
| | | |
| **cut-off** | - **0.4799** | - **0.4799** |

The slightly lower analytical performance in comparison to DxScore1 indicated a model based on 2 biomarkers (miR-23a-3p and piR-5937) (DxScore2). Diagnostic score was calculated according to the following formula :

$$\text{DxScore2} = -0{,}64243 + 39{,}96723*\text{miR-23a-3p} - 1{,}62861*\text{piR-5937}$$

DxScore2 enabled to identify colorectal cancer with sensitivity 94% and specificity 90% in cohort of patients with all TNM stages (Figure 6A,C). If the same DxScore2 and cut-off value was applied in independent validation cohort of patients with early TNM stages of the disease, sensitivity was 82% and specificity 75% (Figure 6B,D), which is still satisfactory performance when compared to current diagnostic approaches used in colorectal cancer (summarized in Table 3).

Table 3 Summary of analytical performance of DxScore2 in diagnosis of colorectal cancer.

| | **Validation phase (TNM stage I-IV)** | **Validation phase (TNM stage I + II)** |
|---|---|---|
| | | |
| **AUC** | 0.9793 | 0.8754 |
| **sensitivity** | 93.67% | 82.01% |
| **specificity** | 90.00% | 75.51% |
| **accuracy** | 91.82% | 78.69% |
| **PPV** | 90.24% | 74.00% |
| **NPV** | 92.31% | 81.82% |
| | | |
| **cut-off** | - **0.09549** | - **0.09549** |

## Claims

1. A method for diagnosing colorectal cancer, **characterized in that** a level of expression of piRNA-hsa-5937, having the sequence TCCCTGGTGGTCTAGTGGTTAGGATTCGGCA (SEQ ID NO. 1), and a level of expression of miR-23a-3p, having the sequence AUCACAUUGCCAGGGAUUUCC (SEQ ID NO. 3), are determined in a sample of body fluid, then the levels of expression of piRNA-hsa-5937 and miR-23a-3p in the sample are compared with levels of expression of piRNA-hsa-5937 and miR-23a-3p in the body fluid of a healthy human, whereas when the level of expression of piRNA-hsa-5937 in the sample is lower than the level of expression of piRNA-hsa-5937 in the body fluid of a healthy human and the level of expression of miR-23a-3p in the sample is higher than the level of expression of miR-23a-3p in body fluid of a healthy human, colorectal cancer is diagnosed in the sample.

2. The method according to claim 1, which further includes determining the levels of expression in the body fluid sample of at least one miRNA selected from:

   miR-27a-3p, having the sequence UUCACAGUGGCUAAGUUCCGC (SEQ ID NO. 4),
   miR-142-5p, having the sequence CAUAAAGUAGAAAGCACUACU (SEQ ID NO. 5),
   comparing them with the levels of expression of the same miRNAs in body fluid of a healthy human, whereas when the the level of expression of said miRNAs in the sample is higher than the level of expression of said miRNAs in body fluid of a healthy human, colorectal cancer is diagnosed in the sample.

3. The method according to any one of the preceding claims, which further includes determining of levels of expression in the body fluid sample of at least one of the following piRNAs, comparing them with the levels of expression of the same piRNA, respectively, in body fluid of a healthy human, whereas when the level of expression of said piRNA shows the change determining colorectal cancer relative to the level of expression of the same piRNA in body fluid of a healthy human, colorectal cancer is diagnosed in the sample:

| piRNA | Sequence | Change in expression level determining colorectal cancer |
|---|---|---|
| piR-hsa-32161 | CCCCCCACTGCTAAATTTGACTGG (SEQ ID NO. 6) | decrease |
| piR-hsa-32238 | CCCCCACTGCTAAATTTGACTGGTT (SEQ ID NO. 7) | decrease |
| piR-hsa-1242 | AGCCCGGCTAGCTCAGTCGGTAGAGCATGAG (SEQ ID NO. 8) | decrease |
| piR-hsa-32187 | CCCCCCACTGCTAAATTTGACTG (SEQ ID NO. 9) | decrease |
| piR-hsa-27620 | GCCCGGCTAGCTCAGTCGGTAGAGCATGAG (SEQ ID NO. 10) | decrease |
| piR-hsa-32162 | CCCCCCACTGCTAAATTTGACTGGC (SEQ ID NO. 11) | decrease |
| piR-hsa-32195 | CCCCCCACTGCTAAATTTGACTGGT (SEQ ID NO. 12) | decrease |
| piR-hsa-32158 | CCCCCCACTGCTAAATTTGACTGGT (SEQ ID NO. 13) | decrease |
| piR-hsa-29218 | TAAAGTGCTGACAGTGCAGATAGTGGTCCTC (SEQ ID NO. 14) | decrease |
| piR-hsa-28876 | GTTTCCGTAGTGTAGTGGTCATCACGTTCGC (SEQ ID NO. 15) | decrease |
| piR-hsa-24672 | TTCCCTGGTGGTCTAGTGGTTAGGATTCGGC (SEQ ID NO. 16) | decrease |
| piR-hsa-26872 | GAGGAATGATGACAAGAAAAGGCCGAA (SEQ ID NO. 17) | increase |
| piR-hsa-8226 | TCTGCTGCCTCAGCCTCCCGAGTAGCTGA (SEQ ID NO. 18) | increase |
| piR-hsa-30715 | TACTCAGGAGGCTGAGGCAGGAGAATGGC (SEQ ID NO. 19) | increase |
| piR-hsa-6746 | TCCTGGGTTCAGGTGATTCTCCTGCCTCAGT (SEQ ID NO. 20) | increase |

(continued)

| piRNA | Sequence | Change in expression level determining colorectal cancer |
|---|---|---|
| piR-hsa-30937 | TAGTCCCAGCTACTTGGGAGGCTGAGGCA (SEQ ID NO. 21) | increase |
| piR-hsa-26543 | GACCAGCCTGGCCAACA TGGTGAAACCCCGCC(SEQ ID NO.22) | increase |
| piR-hsa-15278 | TGCCTCCCGGATTCAAGTGATTCTCCTGCCT (SEQ ID NO. 23) | increase |
| piR-hsa-5505 | TCCCAGCTACCTAGGAGGCTGAGGCAGGAG (SEQ ID NO. 24) | increase |
| piR-hsa-30714 | TACTCAGGAGGCTGAGACAGGAGAATTGC (SEQ ID NO. 25) | increase |
| piR-hsa-1359 | ATCGAGGCTAGAGTCACGCTTGGGTATCGGCT (SEQ ID NO.26) | decrease |
| piR-hsa-27139 | GCCTGGGTAGCTCAGTCGGTAGAGCATCAGA (SEQ ID NO. 27) | decrease |
| piR-hsa-28488 | GGTCGCTGGTTCGAATCCGGCTCGAAGGACC (SEQ ID NO. 28) | decrease |
| piR-hsa-27616 | GCCCGGATGATCCTCAGTGGTCTGGGGTGC (SEQ ID NO. 29) | decrease |
| piR-hsa-29716 | TAAGGTGCATCTAGTGCAGATAGTGAAGTA (SEQ ID NO. 30) | decrease |
| piR-hsa-28019 | GGAGGTGATGAACTGTCTGAGCCTGACC (SEQ ID NO. 31) | decrease |
| piR-hsa-23231 | CCCCTGGTGGTCTAGTGGTTAGGATTCGGC (SEQ ID NO. 32) | decrease |
| piR-hsa-28846 | GTTCACTGATGAGAGCATTGTTCTGAGCCA (SEQ ID NO. 33) | decrease |
| piR-hsa-24360 | TTCACTGATGAGAGCATTGTTCTGAGC (SEQ ID NO. 34) | decrease |
| piR-hsa-6046 | TCCGATCCTCGTTGTTTTGGCTATGGCCAGA (SEQ ID NO. 35) | decrease |

(continued)

| piRNA | Sequence | Change in expression level determining colorectal cancer |
|---|---|---|
| piR-hsa-23940 | CTGACCTCAAGTGATCCACCTGCCTCAGCC (SEQ ID NO. 36) | increase |
| piR-hsa-32182 | CCCCCACTGCTAAATTTGACTGGCTA (SEQ ID NO. 37) | decrease |
| piR-hsa-32167 | CCCCCACTGCTAAATTTGACTGGCT (SEQ ID NO. 38) | decrease |
| piR-hsa-28190 | GGCCGTGATCGTATAGTGGTTAGTACTCTG (SEQ ID NO. 39) | decrease |
| piR-hsa-23210 | CCCCCCACTGCTAAATTTGACTGGTT (SEQ ID NO. 40) | decrease |
| piR-hsa-11362 | TGAGGTAGTAGGTTGTATGGTTTAGAGTTACA (SEQ ID NO.41) | decrease |
| piR-hsa-23209 | CCCCCCACTGCTAAATTTGACTGGCTA (SEQ ID NO. 42) | decrease |
| piR-hsa-32159 | CCCCCCACTGCTAAATTTGACTGGCT (SEQ ID NO. 43) | decrease |

**4.** The method according to any one of the preceding claims, wherein the level of expression of at least one diagnostic marker piRNA or miRNA in the sample is normalized to a substance having a constant content in the body fluid, preferably to miR-93-5p, having the sequence CAAAGUGCUGUUCGUGCAGGUAG (SEQ ID NO. 2).

**5.** The method according to any one of the preceding claims, wherein the level of expression of at least one diagnostic marker piRNA or miRNA in the sample is compared to a pre-determined cut-off value, preferably obtained using a statistical analysis of the levels of expression, optionally normalized levels of expression, in groups of healthy controls and colorectal cancer patients.

**6.** The method according to any one of claims 2-5, wherein the levels of expression of at least two diagnostic marker piRNA or miRNA in the sample are used to calculate a diagnostic score, which is then compared to a pre-determined cut-off value, preferably obtained using a statistical analysis of the levels of expression, optionally normalized levels of expression, in groups of healthy controls and colorectal cancer patients.

**7.** A method of prognosis of overall survival of colorectal cancer patient, **characterized in that** a level of expression of piRNA-hsa-5937 is determined in a sample of body fluid, then the level of expression of piRNA-hsa-5937 is compared with a reference expression level, whereas the level of expression of piRNA-hsa-5937 in the sample which is higher than the reference expression level indicates a favourable overall survival prognosis, while the level of expression of piRNA-hsa-5937 in the sample which is lower than the reference expression level indicates a poor overall survival prognosis, wherein the reference expression level is obtained from a statistical analysis of samples from a group of colorectal cancer patients with favourable overall survival time and a group of colorectal cancer patients with poor overall survival time.

**8.** The method according to claim 7, wherein the level of expression of piRNA-hsa-5937 in the sample is referenced to a substance having a constant level of expression in the body fluid, such as miR-93-5p.

9. The methods according to any one of the preceding claims, wherein the body fluid is selected from blood, blood serum, blood plasma, urine and saliva.

## Patentansprüche

1. Verfahren zur Diagnose von Darmkrebs, **dadurch gekennzeichnet, dass** ein Expressionslevel von piRNA-hsa-5937 mit der Sequenz TCCCTGGTGGTCTAGTGGTTAGGATTCGGCA (SEQ ID NO. 1) und ein Expressionslevel von miR-23a-3p mit der Sequenz AUCACAUUGCCAGGGAUUUCC (SEQ ID NO. 3) werden in einer Probe von Körperflüssigkeit bestimmt, dann werden die Expressionslevels von piRNA-hsa-5937 und miR-23a-3p in der Probe mit Expressionslevels von piRNA-hsa-5937 und miR-23a-3p in der Körperflüssigkeit eines gesunden Menschen verglichen, und wenn das Expressionslevel von piRNA-hsa-5937 in der Probe niedriger ist als das Expressionslevel von piRNA-hsa-5937 in der Körperflüssigkeit eines gesunden Menschen, und das Expressionslevel von miR-23a-3p in der Probe höher ist als das Expressionslevel von miR-23a-3p in der Körperflüssigkeit eines gesunden Menschen, wird Darmkrebs in der Probe diagnostiziert.

2. Verfahren nach Anspruch 1, **dadurch gekennzeichnet, dass** in der Körperflüssigkeitsprobe auch die Expressionslevels mindestens einer miRNA bestimmt werden, ausgewählt aus:

miR-27a-3p mit der Sequenz UUCACAGUGGCUAAGUUCCGC (SEQ ID NO. 4),
miR-142-5p mit der Sequenz CAUAAAGUAGAAAGCACUACU (SEQ ID NO. 5),
und verglichen werden mit den Expressionslevels derselben miRNAs in Körperflüssigkeit eines gesunden Menschen, und wenn das Expressionslevel dieser miRNAs in der Probe höher ist als das Expressionslevel dieser miRNAs in Körperflüssigkeit eines gesunden Menschen, Darmkrebs wird in der Probe diagnostiziert.

3. Verfahren nach einem der vorhergehenden Ansprüche, bei dem ferner die Expressionslevels in der Körperflüssigkeitsprobe von mindestens einer der folgenden piRNAs bestimmt und mit den Expressionslevels derselben piRNA in Körperflüssigkeit eines gesunden Menschen verglichen werden, und wenn das Expressionslevel der piRNA die Veränderung zeigt im Verhältnis zum Expressionslevel der gleichen piRNA in der Körperflüssigkeit eines gesunden Menschen die Darmkrebs bestimmt, Darmkrebs wird in der Probe diagnostiziert:

| **piRNA** | **Sequenz** | **Veränderung des Expressionslevels zur Bestimmung von Darmkrebs** |
|---|---|---|
| piR-hsa-32161 | CCCCCCACTGCTAAATTTGACTGG (SEQ ID NO. 6) | verringern |
| piR-hsa-32238 | CCCCCACTGCTAAATTTGACTGGTT (SEQ ID NO. 7) | verringern |
| piR-hsa-1242 | AGCCCGGCTAGCTCAGTCGGTAGAGCATGAG (SEQ ID NO. 8) | verringern |
| piR-hsa-32187 | CCCCCCACTGCTAAATTTGACTG (SEQ ID NO. 9) | verringern |
| piR-hsa-27620 | GCCCGGCTAGCTCAGTCGGTAGAGCATGAG (SEQ ID NO. 10) | verringern |
| piR-hsa-32162 | CCCCCCACTGCTAAATTTGACTGGC (SEQ ID NO. 11) | verringern |

(fortgesetzt)

| piRNA | Sequenz | Veränderung des Expressionslevels zur Bestimmung von Darmkrebs |
|---|---|---|
| piR-hsa-32195 | CCCCCCACTGCTAAATTTGACTGGT (SEQ ID NO. 12) | verringern |
| piR-hsa-32158 | CCCCCCACTGCTAAATTTGACTGGT (SEQ ID NO. 13) | verringern |
| piR-hsa-29218 | TAAAGTGCTGACAGTGCAGATAGTGGTCCTC (SEQ ID NO. 14) | verringern |
| piR-hsa-28876 | GTTTCCGTAGTGTAGTGGTCATCACGTTCGC (SEQ ID NO. 15) | verringern |
| piR-hsa-24672 | TTCCCTGGTGGTCTAGTGGTTAGGATTCGGC (SEQ ID NO. 16) | verringern |
| piR-hsa-26872 | GAGGAATGATGACAAGAAAAGGCCGAA (SEQ ID NO. 17) | erhöhen |
| piR-hsa-8226 | TCTGCTGCCTCAGCCTCCCGAGTAGCTGA (SEQ ID NO. 18) | erhöhen |
| piR-hsa-30715 | TACTCAGGAGGCTGAGGCAGGAGAATGGC (SEQ ID NO. 19) | erhöhen |
| piR-hsa-6746 | TCCTGGGTTCAGGTGATTCTCCTGCCTCAGT (SEQ ID NO. 20) | erhöhen |
| piR-hsa-30937 | TAGTCCCAGCTACTTGGGAGGCTGAGGCA (SEQ ID NO. 21) | erhöhen |
| piR-hsa-26543 | GACCAGCCTGGCCAACATGGTGAAACCCCGCC (SEQ ID NO.22) | erhöhen |
| piR-hsa-15278 | TGCCTCCCGGATTCAAGTGATTCTCCTGCCT(SEQ ID NO. 23) | erhöhen |
| piR-hsa-5505 | TCCCAGCTACCTAGGAGGCTGAGGCAGGAG (SEQ ID NO. 24) | erhöhen |
| piR-hsa-30714 | TACTCAGGAGGCTGAGACAGGAGAATTGC (SEQ ID NO. 25) | erhöhen |
| piR-hsa-1359 | ATCGAGGCTAGAGTCACGCTTGGGTATCGGCT(SEQ ID NO.26) | verringern |

(fortgesetzt)

| piRNA | Sequenz | Veränderung des Expressionslevels zur Bestimmung von Darmkrebs |
|---|---|---|
| piR-hsa-27139 | GCCTGGGTAGCTCAGTCGGTAGAGCATCAGA (SEQ ID NO. 27) | verringern |
| piR-hsa-28488 | GGTCGCTGGTTCGAATCCGGCTCGAAGGACC (SEQ ID NO. 28) | verringern |
| piR-hsa-27616 | GCCCGGATGATCCTCAGTGGTCTGGGGTGC (SEQ ID NO. 29) | verringern |
| piR-hsa-29716 | TAAGGTGCATCTAGTGCAGATAGTGAAGTA (SEQ ID NO. 30) | verringern |
| piR-hsa-28019 | GGAGGTGATGAACTGTCTGAGCCTGACC (SEQ ID NO. 31) | verringern |
| piR-hsa-23231 | CCCCTGGTGGTCTAGTGGTTAGGATTCGGC (SEQ ID NO. 32) | verringern |
| piR-hsa-28846 | GTTCACTGATGAGAGCATTGTTCTGAGCCA (SEQ ID NO. 33) | verringern |
| piR-hsa-24360 | TTCACTGATGAGAGCATTGTTCTGAGC (SEQ ID NO. 34) | verringern |
| piR-hsa-6046 | TCCGATCCTCGTTGTTTTGGCTATGGCCAGA (SEQ ID NO. 35) | verringern |
| piR-hsa-23940 | CTGACCTCAAGTGATCCACCTGCCTCAGCC (SEQ ID NO. 36) | erhöhen |
| piR-hsa-32182 | CCCCCACTGCTAAATTTGACTGGCTA (SEQ ID NO. 37) | verringern |
| piR-hsa-32167 | CCCCCACTGCTAAATTTGACTGGCT (SEQ ID NO. 38) | verringern |
| piR-hsa-28190 | GGCCGTGATCGTATAGTGGTTAGTACTCTG (SEQ ID NO. 39) | verringern |
| piR-hsa-23210 | CCCCCCACTGCTAAATTTGACTGGTT (SEQ ID NO. 40) | verringern |
| piR-hsa-11362 | TGAGGTAGTAGGTTGTATGGTTTAGAGTTACA (SEQ ID NO.41) | verringern |

(fortgesetzt)

| piRNA | Sequenz | Veränderung des Expressionslevels zur Bestimmung von Darmkrebs |
|---|---|---|
| piR-hsa-23209 | CCCCCCACTGCTAAATTTGACTGGCTA (SEQ ID NO. 42) | verringern |
| piR-hsa-32159 | CCCCCCACTGCTAAATTTGACTGGCT (SEQ ID NO. 43) | verringern |

**4.** Verfahren nach einem der vorhergehenden Ansprüche, wobei das Expressionslevel von mindestens einem diagnostischen piRNA oder miRNA Marker in der Probe auf eine Substanz mit einem konstanten Gehalt in der Körperflüssigkeit, vorzugsweise auf miR-93-5p mit der Sequenz CAAAGUGCUGUUCGUGCAGGUAG (SEQ ID NO. 2), normalisiert wird.

**5.** Verfahren nach einem der vorhergehenden Ansprüche, wobei das Expressionslevel von mindestens einem diagnostischen piRNA oder miRNA Marker in der Probe mit einem vorbestimmten Cut-Off-Wert verglichen wird, der vorzugsweise unter Verwendung einer statistischen Analyse von Expressionslevels, gegebenenfalls normalisierter Expressionslevels, in Gruppen von gesunden Kontrollpersonen und Patienten mit Darmkrebs erhalten wird.

**6.** Verfahren nach einem der Ansprüche 2 bis 5, wobei die Expressionslevels von mindestens zwei diagnostischen piRNA oder miRNA Markern in der Probe verwendet werden, um einen diagnostischen Score zu berechnen, der dann mit einem vorbestimmten Cut-Off-Wert verglichen wird, das vorzugsweise erhalten wird unter Verwendung einer statistischen Analyse der Expressionslevels, gegebenenfalls normalisierter Expressionslevels, in Gruppen von gesunden Kontrollpersonen und Patienten mit Darmkrebs.

**7.** Verfahren zur Prognose des Gesamtüberlebens eines Darmkrebspatienten, **dadurch gekennzeichnet, dass** ein Expressionslevel von piRNA-hsa-5937 in einer Körperflüssigkeitsprobe bestimmt wird und dann das Expressionslevel von piRNA-hsa-5937 verglichen wird mit einem Referenz-Expressionslevel, und wenn das Expressionslevel von piRNA-hsa-5937 in der Probe höher als das Referenz-Expressionslevel ist, eine günstige Gesamtüberlebensprognose abgeschlossen ist, weil wenn das Expressionslevel von piRNA-hsa-5937 in der Probe niedriger als das Referenz-Expressionslevel ist, eine schlechte Gesamtüberlebensprognose abgeschlossen ist, wobei das Referenz-Expressionslevel aus einer statistischen Analyse von Proben einer Gruppe von Darmkrebspatienten mit günstiger Gesamtüberlebenszeit und einer Gruppe von Darmkrebspatienten mit schlechter Gesamtüberlebenszeit erhalten wird.

**8.** Verfahren nach Anspruch 7, wobei das Expressionslevel von piRNA-hsa-5937 in der Probe auf eine Substanz mit einem konstanten Expressionslevel in der Körperflüssigkeit, wie miR-93-5p, bezogen wird.

**9.** Verfahren nach einem der vorhergehenden Ansprüche, wobei die Körperflüssigkeit ausgewählt ist aus Blut, Blutserum, Blutplasma, Urin und Speichel.

## Revendications

**1.** Procédé de diagnostic du cancer colorectal, **caractérisé en ce qu'**un niveau d'expression de piRNA-hsa-5937, ayant la séquence TCCCTGGTGGTCTAGTGGTTAGGATTCGGCA (SEQ ID NO. 1), et un niveau d'expression de miR-23a-3p, ayant la séquence AUCACAUUGCCAGGGAUUUCC (SEQ ID NO. 3), sont déterminés dans un échantillon de liquide corporel, puis les niveaux d'expression de piRNA-hsa-5937 et miR-23a-3p dans l'échantillon sont comparés aux niveaux d'expression de piRNA-hsa-5937 et miR-23a-3p dans le liquide corporel d'un humain sain, alors que lorsque le niveau d'expression de piRNA-hsa-5937 dans l'échantillon est diminué par rapport au niveau d'expression de piRNA-hsa-5937 dans le liquide corporel d'un humain sain et le niveau d'expression de miR-23a-3p dans l'échantillon est augmenté par rapport au niveau d'expression de miR-23a-3p dans le liquide corporel d'un humain sain, un cancer colorectal est diagnostiqué dans l'échantillon.

**2.** Procédé selon la revendication 1, qui comprend en outre la détermination des niveaux d'expression dans l'échantillon de liquide corporel d'au moins un miRNA choisi parmi:

miR-27a-3p, ayant la séquence UUCACAGUGGCUAAGUUCCGC (SEQ ID NO. 4),
miR-142-5p, ayant la séquence CAUAAAGUAGAAAGCACUACU (SEQ ID NO. 5),
les comparer aux niveaux d'expression des mêmes miRNAs dans le liquide corporel d'un humain sain, alors que lorsque le niveau d'expression desdits miRNAs dans l'échantillon est augmenté par rapport au niveau d'expression desdits miRNAs dans le liquide corporel d'un humain sain, le cancer colorectal est diagnostiqué dans l'échantillon.

**3.** Procédé selon l'une quelconque des revendications précédentes, qui comprend en outre la détermination des niveaux d'expression dans l'échantillon de liquide corporel d'au moins l'un des piRNAs suivants, en les comparant avec les niveaux d'expression du même piRNA, respectivement, dans le liquide corporel d'un humain sain, alors que lorsque le niveau d'expression dudit piRNA montre le changement déterminant le cancer colorectal par rapport au niveau d'expression du même piRNA dans le liquide corporel d'un humain sain, le cancer colorectal est diagnostiqué dans l'échantillon:

| **piRNA** | **Séquence** | **Changement du niveau d'expression déterminant le cancer colorectal** |
|---|---|---|
| piR-hsa-32161 | CCCCCCACTGCTAAATTTGACTGG (SEQ ID NO. 6) | diminution |
| piR-hsa-32238 | CCCCCACTGCTAAATTTGACTGGTT (SEQ ID NO. 7) | diminution |
| piR-hsa-1242 | AGCCCGGCTAGCTCAGTCGGTAGAGCATGAG (SEQ ID NO. 8) | diminution |
| piR-hsa-32187 | CCCCCCACTGCTAAATTTGACTG (SEQ ID NO. 9) | diminution |
| piR-hsa-27620 | GCCCGGCTAGCTCAGTCGGTAGAGCATGAG (SEQ ID NO. 10) | diminution |
| piR-hsa-32162 | CCCCCCACTGCTAAATTTGACTGGC (SEQ ID NO. 11) | diminution |
| piR-hsa-32195 | CCCCCCACTGCTAAATTTGACTGGT (SEQ ID NO. 12) | diminution |
| piR-hsa-32158 | CCCCCCACTGCTAAATTTGACTGGT (SEQ ID NO. 13) | diminution |
| piR-hsa-29218 | TAAAGTGCTGACAGTGCAGATAGTGGTCCTC (SEQ ID NO. 14) | diminution |
| piR-hsa-28876 | GTTTCCGTAGTGTAGTGGTCATCACGTTCGC (SEQ ID NO. 15) | diminution |

(suite)

| piRNA | Séquence | Changement du niveau d'expression déterminant le cancer colorectal |
|---|---|---|
| piR-hsa-24672 | TTCCCTGGTGGTCTAGTGGTTAGGATTCGGC (SEQ ID NO. 16) | diminution |
| piR-hsa-26872 | GAGGAATGATGACAAGAAAAGGCCGAA (SEQ ID NO. 17) | augmentation |
| piR-hsa-8226 | TCTGCTGCCTCAGCCTCCCGAGTAGCTGA (SEQ ID NO. 18) | augmentation |
| piR-hsa-30715 | TACTCAGGAGGCTGAGGCAGGAGAATGGC (SEQ ID NO. 19) | augmentation |
| piR-hsa-6746 | TCCTGGGTTCAGGTGATTCTCCTGCCTCAGT (SEQ ID NO. 20) | augmentation |
| piR-hsa-30937 | TAGTCCCAGCTACTTGGGAGGCTGAGGCA (SEQ ID NO. 21) | augmentation |
| piR-hsa-26543 | GACCAGCCTGGCCAACATGGTGAAACCCCGCC(SEQ ID NO. 22) | augmentation |
| piR-hsa-15278 | TGCCTCCCGGATTCAAGTGATTCTCCTGCCT (SEQ ID NO. 23) | augmentation |
| piR-hsa-5505 | TCCCAGCTACCTAGGAGGCTGAGGCAGGAG (SEQ ID NO. 24) | augmentation |
| piR-hsa-30714 | TACTCAGGAGGCTGAGACAGGAGAATTGC (SEQ ID NO. 25) | augmentation |
| piR-hsa-1359 | ATCGAGGCTAGAGTCACGCTTGGGTATCGGCT (SEQ ID NO. 26) | diminution |
| piR-hsa-27139 | GCCTGGGTAGCTCAGTCGGTAGAGCATCAGA (SEQ ID NO. 27) | diminution |
| piR-hsa-28488 | GGTCGCTGGTTCGAATCCGGCTCGAAGGACC (SEQ ID NO. 28) | diminution |
| piR-hsa-27616 | GCCCGGATGATCCTCAGTGGTCTGGGGTGC (SEQ ID NO. 29) | diminution |
| piR-hsa-29716 | TAAGGTGCATCTAGTGCAGATAGTGAAGTA (SEQ ID NO. 30) | diminution |

(suite)

| piRNA | Séquence | Changement du niveau d'expression déterminant le cancer colorectal |
|---|---|---|
| piR-hsa-28019 | GGAGGTGATGAACTGTCTGAGCCTGACC (SEQ ID NO. 31) | diminution |
| piR-hsa-23231 | CCCCTGGTGGTCTAGTGGTTAGGATTCGGC (SEQ ID NO. 32) | diminution |
| piR-hsa-28846 | GTTCACTGATGAGAGCATTGTTCTGAGCCA (SEQ ID NO. 33) | diminution |
| piR-hsa-24360 | TTCACTGATGAGAGCATTGTTCTGAGC (SEQ ID NO. 34) | diminution |
| piR-hsa-6046 | TCCGATCCTCGTTGTTTTGGCTATGGCCAGA (SEQ ID NO. 35) | diminution |
| piR-hsa-23940 | CTGACCTCAAGTGATCCACCTGCCTCAGCC (SEQ ID NO. 36) | augmentation |
| piR-hsa-32182 | CCCCCACTGCTAAATTTGACTGGCTA (SEQ ID NO. 37) | diminution |
| piR-hsa-32167 | CCCCCACTGCTAAATTTGACTGGCT (SEQ ID NO. 38) | diminution |
| piR-hsa-28190 | GGCCGTGATCGTATAGTGGTTAGTACTCTG (SEQ ID NO. 39) | diminution |
| piR-hsa-23210 | CCCCCCACTGCTAAATTTGACTGGTT (SEQ ID NO. 40) | diminution |
| piR-hsa-11362 | TGAGGTAGTAGGTTGTATGGTTTAGAGTTACA (SEQ ID NO. 41) | diminution |
| piR-hsa-23209 | CCCCCCACTGCTAAATTTGACTGGCTA (SEQ ID NO. 42) | diminution |
| piR-hsa-32159 | CCCCCCACTGCTAAATTTGACTGGCT (SEQ ID NO. 43) | diminution |

**4.** Procédé selon l'une quelconque des revendications précédentes, dans lequel le niveau d'expression d'au moins un piRNA ou miRNA, agissant comme marqueur diagnostique, dans l'échantillon est normalisé à une substance ayant une teneur constante dans le liquide corporel, de préférence à miR-93-5p, ayant la séquence CAAAGUGCUGUUCGUGCAGGUAG (SEQ ID NO. 2).

**5.** Procédé selon l'une quelconque des revendications précédentes, dans lequel le niveau d'expression d'au moins un piRNA ou miRNA, agissant comme marqueur diagnostique, dans l'échantillon est comparé à une valeur seuil prédéterminée, obtenue de préférence en utilisant une analyse statistique des niveaux d'expression, ou éventuellement des niveaux d'expression normalisés, dans des groupes de humains sains et des patients atteints de cancer colorectal.

**6.** Procédé selon l'une quelconque des revendications 2 à 5, dans lequel les niveaux d'expression d'au moins deux marqueurs diagnostiques piRNA ou miRNA dans l'échantillon sont utilisés pour calculer un score diagnostique, qui est ensuite comparé à une valeur seuil prédéterminée, de préférence obtenue en utilisant une analyse statistique des niveaux d'expression, ou éventuellement des niveaux d'expression normalisés, dans des groupes de humains sains et de patients atteints de cancer colorectal.

**7.** Procédé de pronostic de survie globale d'un patient atteint d'un cancer colorectal, **caractérisé en ce qu'**un niveau d'expression de piRNA-hsa-5937 est déterminé dans un échantillon de liquide corporel, puis le niveau d'expression de piRNA-hsa-5937 est comparé à un niveau d'expression de piRNA-hsa-5937 de référence, tandis que le niveau d'expression de piRNA-hsa-5937 dans l'échantillon qui est augmenté par rapport au niveau d'expression de référence indique un pronostic de survie globale favorable, tandis que le niveau d'expression de piRNA-hsa-5937 dans l'échantillon qui est diminué par rapport au niveau d'expression de référence indique un mauvais pronostic de survie globale, le niveau d'expression de référence étant obtenu à partir d'une analyse statistique d'échantillons provenant d'un groupe de patients atteints de cancer colorectal avec un temps de survie globale favorable et d'un groupe de patients atteints de cancer colorectal ayant un mauvais temps de survie globale .

**8.** Procédé selon la revendication 7, dans lequel le niveau d'expression de piRNA-hsa-5937 dans l'échantillon est normalisé à une substance ayant un niveau d'expression constant dans le liquide corporel, tel que miR-93-5p.

**9.** Procédé selon l'une quelconque des revendications précédentes, dans lequel le liquide corporel est choisi parmi le sang, le sérum sanguin, le plasma sanguin, l'urine et la salive.

Figure 1

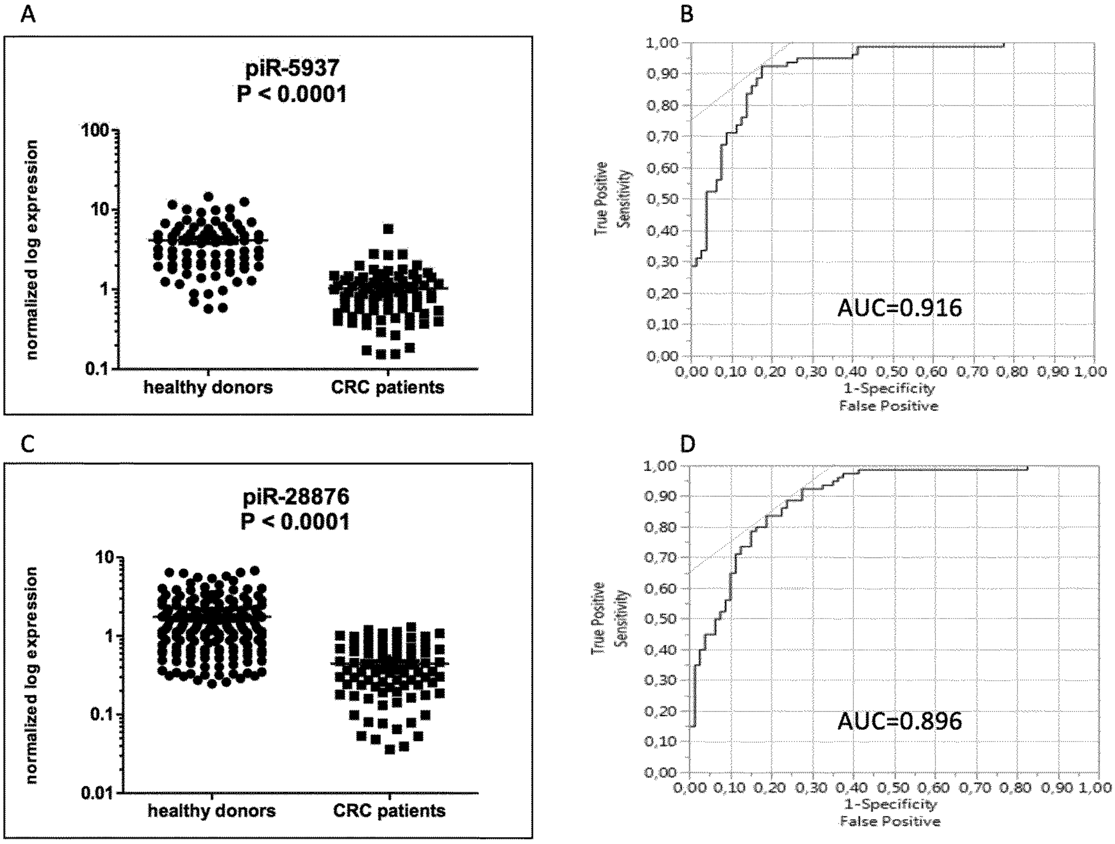


Figure 2

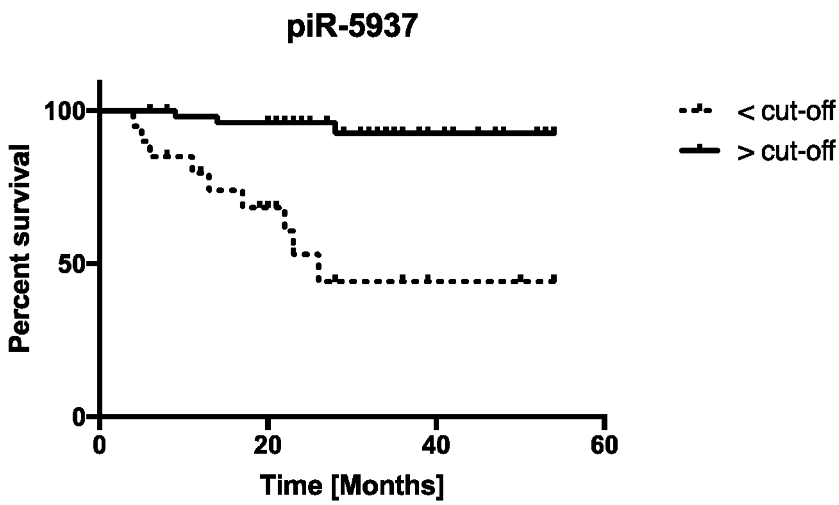

Figure 3

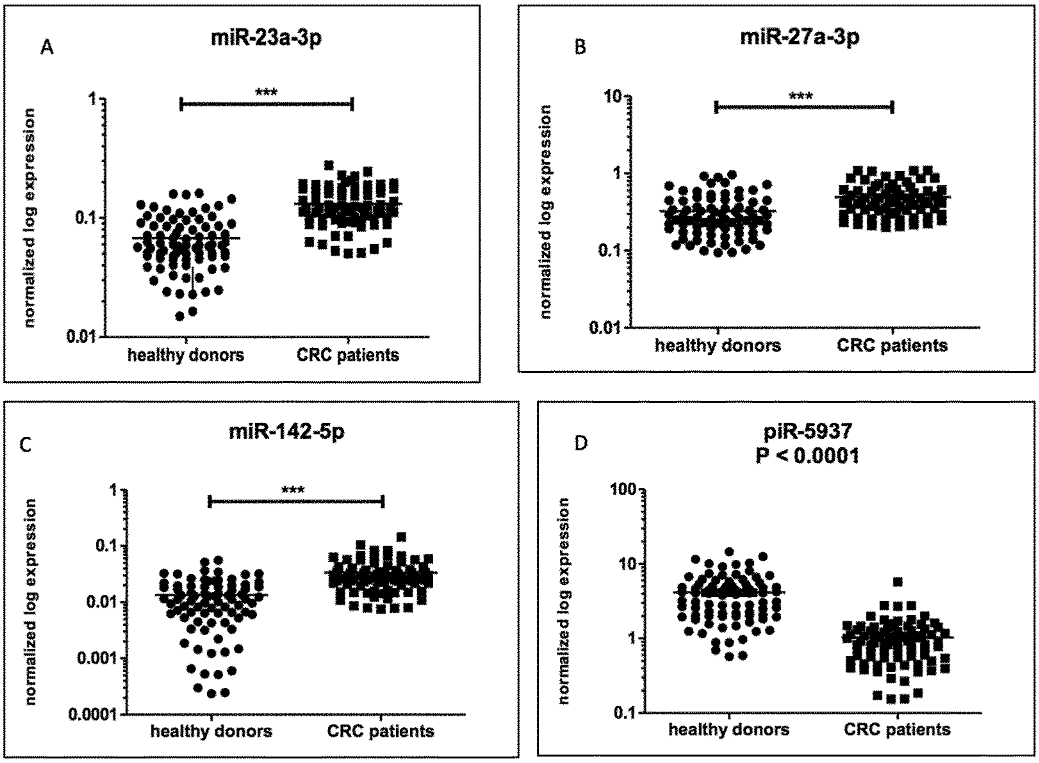


Figure 4

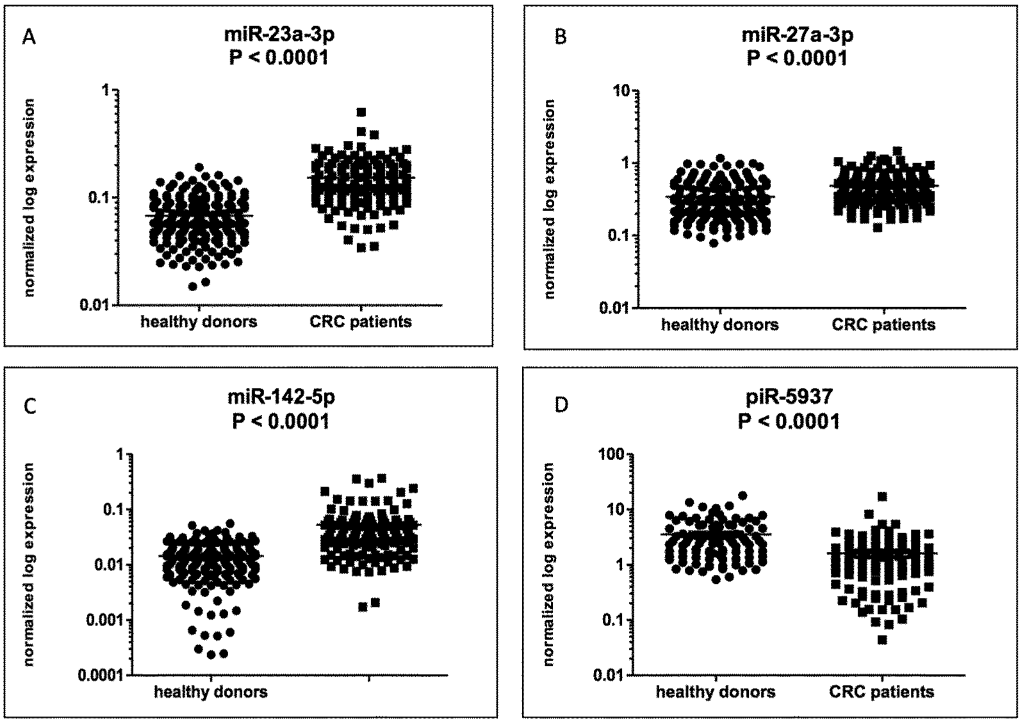

Figure 5

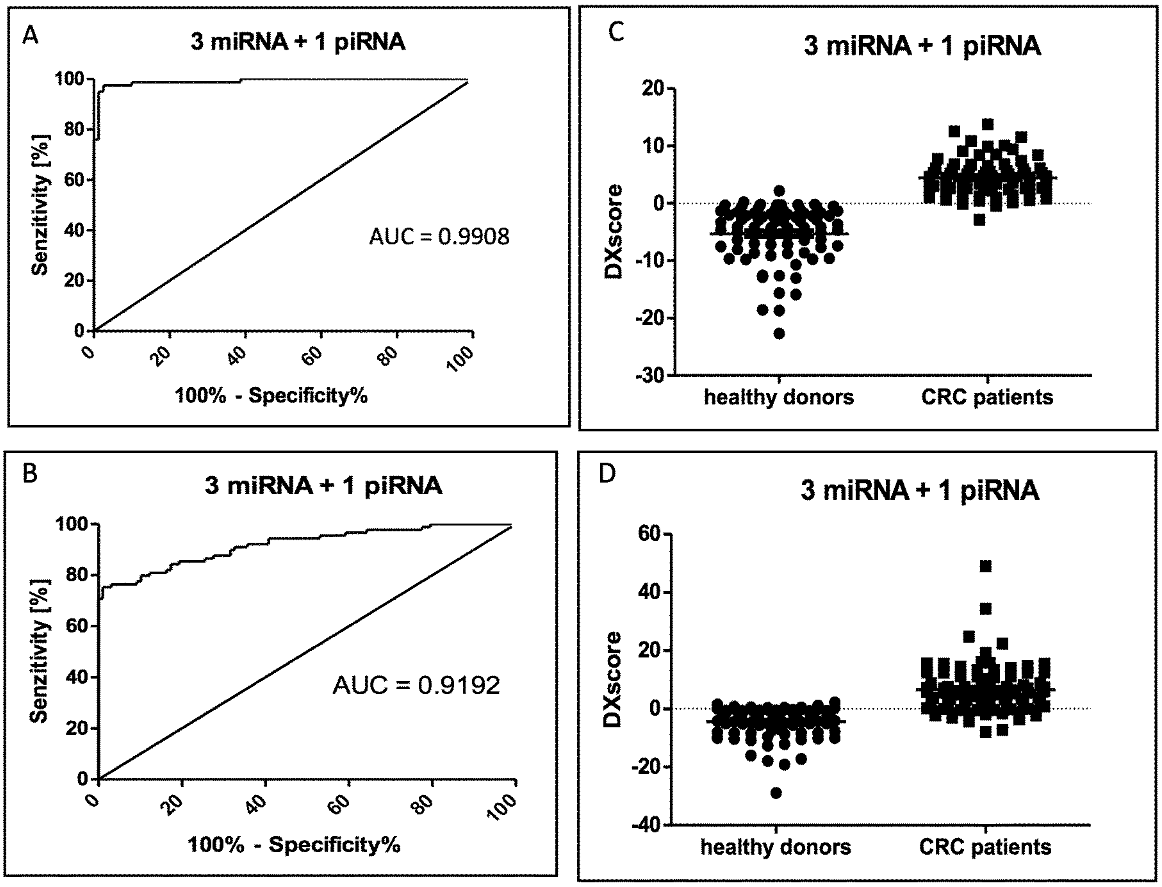
A
3 miRNA + 1 piRNA
Senzitivity [%]
100% - Specificity%
AUC = 0.9908
B
3 miRNA + 1 piRNA
Senzitivity [%]
100% - Specificity%
AUC = 0.9192
C
3 miRNA + 1 piRNA
DXscore
healthy donors
CRC patients
D
3 miRNA + 1 piRNA
DXscore
healthy donors
CRC patients

Figure 6

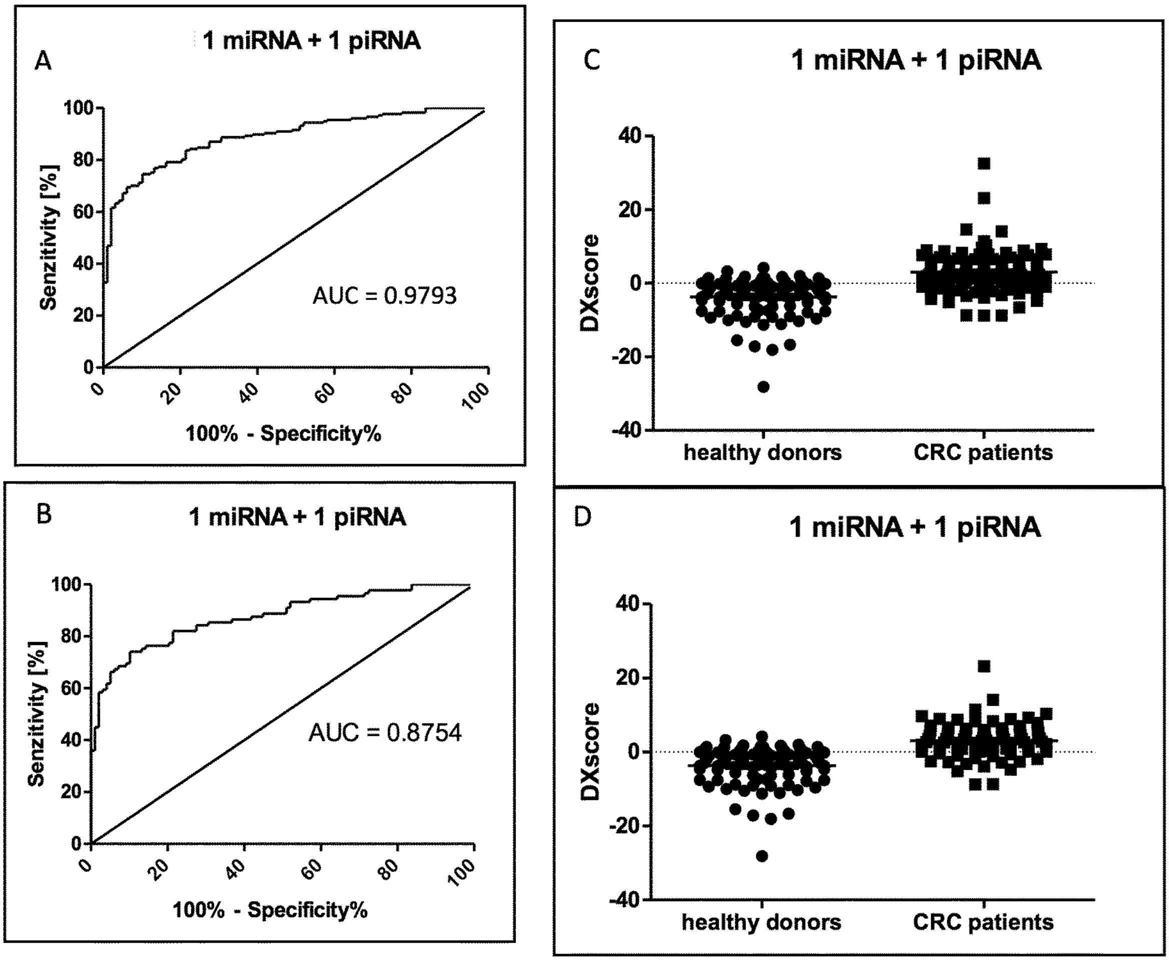
A
1 miRNA + 1 piRNA
Senzitivity [%]
100
80
60
40
20
0
AUC = 0.9793
0
20
40
60
80
100
100% - Specificity%
B
1 miRNA + 1 piRNA
Senzitivity [%]
AUC = 0.8754
100% - Specificity%
C
1 miRNA + 1 piRNA
DXscore
40
20
0
-20
-40
healthy donors
CRC patients
D
1 miRNA + 1 piRNA
DXscore
healthy donors
CRC patients

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010061996 A1
- WO 2005015224 A
- WO 2004079368 A
- WO 2004071267 A
- WO 2005015234 A
- US 7501243 B


### Non-patent literature cited in the description


- **CRC. WARREN et al.** *BMC Med,* 2011, vol. 9, 133
- **CRC. BRUENAGEL et al.** *Cancer Research,* 2002, vol. 62, 2437-2442
- **VYCHYTILOVA-FALTEJSKOVA et al.** *Carcinogenesis,* 2016, 941-950
- **OZAWA et al.** *Gastroenterology,* 2017, vol. 152 (5), 152

SEQUENCE LISTING

<110> Masarykova univerzita

<120> Method of diagnosis of colorectal cancer

<130> P

<160> 43

<170> PatentIn version 3.5

<210> 1
<211> 31
<212> DNA
<213> homo sapiens

<400> 1
tccctggtgg tctagtggtt aggattcggc a 31

<210> 2
<211> 23
<212> RNA
<213> homo sapiens

<400> 2
caaagugcug uucgugcagg uag 23

<210> 3
<211> 21
<212> DNA
<213> homo sapiens

<400> 3
aucacauugc cagggauuuc c 21

<210> 4
<211> 21
<212> DNA
<213> homo sapiens

<400> 4
uucacagugg cuaaguuccg c 21

<210> 5
<211> 21
<212> DNA
<213> homo sapiens

<400> 5
cauaaaguag aaagcacuac u 21

<210> 6
<211> 24
<212> DNA
<213> homo sapiens

<400> 6
ccccccactg ctaaatttga ctgg 24

<210> 7
<211> 25
<212> DNA
<213> homo sapiens

<400> 7
cccccactgc taaatttgac tggtt 25

<210> 8
<211> 31
<212> DNA
<213> homo sapiens

<400> 8
agcccggcta gctcagtcgg tagagcatga g 31

<210> 9
<211> 23
<212> DNA
<213> homo sapiens

<400> 9
ccccccactg ctaaatttga ctg 23

<210> 10
<211> 30
<212> DNA
<213> homo sapiens

<400> 10
gcccggctag ctcagtcggt agagcatgag 30

<210> 11
<211> 25
<212> DNA
<213> homo sapiens

<400> 11
ccccccactg ctaaatttga ctggc 25

<210> 12
<211> 25
<212> DNA
<213> homo sapiens

<400> 12
ccccccactg ctaaatttga ctggt 25

<210> 13
<211> 25
<212> DNA
<213> homo sapiens

<400> 13
ccccccactg ctaaatttga ctggt 25

<210> 14
<211> 31
<212> DNA
<213> homo sapiens

<400> 14
taaagtgctg acagtgcaga tagtggtcct c 31

<210> 15
<211> 31
<212> DNA
<213> homo sapiens

<400> 15
gtttccgtag tgtagtggtc atcacgttcg c 31

<210> 16
<211> 31
<212> DNA
<213> homo sapiens

<400> 16
ttccctggtg gtctagtggt taggattcgg c 31

<210> 17
<211> 27
<212> DNA
<213> homo sapiens

<400> 17
gaggaatgat gacaagaaaa ggccgaa 27

<210> 18
<211> 29

<212> DNA
<213> homo sapiens

<400> 18
tctgctgcct cagcctcccg agtagctga 29

<210> 19
<211> 29
<212> DNA
<213> homo sapiens

<400> 19
tactcaggag gctgaggcag gagaatggc 29

<210> 20
<211> 31
<212> DNA
<213> homo sapiens

<400> 20
tcctgggttc aggtgattct cctgcctcag t 31

<210> 21
<211> 29
<212> DNA
<213> homo sapiens

<400> 21
tagtcccagc tacttgggag gctgaggca 29

<210> 22
<211> 32
<212> DNA
<213> homo sapiens

<400> 22
gaccagcctg gccaacatgg tgaaaccccg cc 32

<210> 23
<211> 31
<212> DNA
<213> homo sapiens

<400> 23
tgcctcccgg attcaagtga ttctcctgcc t 31

<210> 24
<211> 30
<212> DNA
<213> homo sapiens

<400> 24
tcccagctac ctaggaggct gaggcaggag 30

<210> 25
<211> 29
<212> DNA
<213> homo sapiens

<400> 25
tactcaggag gctgagacag gagaattgc 29

<210> 26
<211> 32
<212> DNA
<213> homo sapiens

<400> 26
atcgaggcta gagtcacgct tgggtatcgg ct 32

<210> 27
<211> 31
<212> DNA
<213> homo sapiens

<400> 27
gcctgggtag ctcagtcggt agagcatcag a 31

<210> 28
<211> 31
<212> DNA
<213> homo sapiens

<400> 28
ggtcgctggt tcgaatccgg ctcgaaggac c 31

<210> 29
<211> 30
<212> DNA
<213> homo sapiens

<400> 29
gcccggatga tcctcagtgg tctggggtgc 30

<210> 30
<211> 30
<212> DNA
<213> homo sapiens

<400> 30
taaggtgcat ctagtgcaga tagtgaagta 30

<210> 31
<211> 28
<212> DNA
<213> homo sapiens

<400> 31
ggaggtgatg aactgtctga gcctgacc 28

<210> 32
<211> 30
<212> DNA
<213> homo sapiens

<400> 32
cccctggtgg tctagtggtt aggattcggc 30

<210> 33
<211> 30
<212> DNA
<213> homo sapiens

<400> 33
gttcactgat gagagcattg ttctgagcca 30

<210> 34
<211> 27
<212> DNA
<213> homo sapiens

<400> 34
ttcactgatg agagcattgt tctgagc 27

<210> 35
<211> 31
<212> DNA
<213> homo sapiens

<400> 35
tccgatcctc gttgttttgg ctatggccag a 31

<210> 36
<211> 30
<212> DNA
<213> homo sapiens

<400> 36
ctgacctcaa gtgatccacc tgcctcagcc 30

<210> 37
<211> 26
<212> DNA
<213> homo sapiens

<400> 37
cccccactgc taaatttgac tggcta 26

<210> 38
<211> 25
<212> DNA
<213> homo sapiens

<400> 38
cccccactgc taaatttgac tggct 25

<210> 39
<211> 30
<212> DNA
<213> homo sapiens

<400> 39
ggccgtgatc gtatagtggt tagtactctg 30

<210> 40
<211> 26

<212> DNA
<213> homo sapiens

<400> 40
ccccccactg ctaaatttga ctggtt 26

<210> 41
<211> 32
<212> DNA
<213> homo sapiens

<400> 41
tgaggtagta ggttgtatgg tttagagtta ca 32

<210> 42
<211> 27
<212> DNA
<213> homo sapiens

<400> 42
ccccccactg ctaaatttga ctggcta 27

<210> 43
<211> 26
<212> DNA
<213> homo sapiens

<400> 43
ccccccactg ctaaatttga ctggct 26